(19) Europäisches Patentamt
European Patent Office
Office européen des brevets

(11) EP 1 399 439 B1

(12) EUROPEAN PATENT SPECIFICATION

(45) Date of publication and mention of the grant of the patent:
**14.06.2006 Bulletin 2006/24**

(21) Application number: **02730265.2**

(22) Date of filing: **21.05.2002**

(51) Int Cl.:
*C07D 409/04* (2006.01) *C07D 417/04* (2006.01)
*C07D 401/04* (2006.01) *C07D 407/04* (2006.01)
*C07D 403/04* (2006.01) *C07D 487/04* (2006.01)
*A61K 31/53* (2006.01) *A61P 37/00* (2006.01)
*A61P 29/00* (2006.01) *A61P 11/00* (2006.01)
*A61P 11/06* (2006.01)

(86) International application number:
**PCT/EP2002/005540**

(87) International publication number:
**WO 2002/098873 (12.12.2002 Gazette 2002/50)**

(54) **2-HETEROARYL-IMIDAZOTRIAZINONES AND THEIR USE IN THE TREATMENT OF INFLAMMATORY OR IMMUNE DISEASES**

2-HETEROARYL-IMIDAZOTRIAZINONE UND IHRE VERWENDUNG BEI DER BEHANDLUNG VON ENTZÜNDUNGS- ODER IMMUNERKRANKUNGEN

2-HETEROARYL-IMIDAZOTRIAZINONES ET UTILISATION DANS LE TRAITEMENT DE MALADIES INFLAMMATOIRES OU IMMUNITAIRES

(84) Designated Contracting States:
**DE ES FR GB IT**

(30) Priority: **01.06.2001 GB 0113343**

(43) Date of publication of application:
**24.03.2004 Bulletin 2004/13**

(73) Proprietor: **Bayer HealthCare AG**
**51368 Leverkusen (DE)**

(72) Inventors:
- **ALONSO-ALIJA, Cristina**
  **42781 Haan (DE)**
- **GIELEN, Heike**
  **51379 Leverkusen (DE)**
- **HENDRIX, Martin**
  **51519 Odenthal (DE)**
- **NIEWÖHNER, Ulrich**
  **verstorben (DE)**
- **SCHAUSS, Dagmar**
  **42697 Solingen (DE)**
- **BISCHOFF, Hilmar**
  **42113 Wuppertal (DE)**
- **BURKHARDT, Nils**
  **42553 Velbert (DE)**
- **GEISS, Volker**
  **40883 Ratingen (DE)**
- **SCHLEMMER, Karl-Heinz**
  **42113 Wuppertal (DE)**
- **CUTHBERT,Nigel**
  **Tiverton Devon EX 16 7AE (GB)**
- **Cuthbert, Nigel**
  **Hogshaw, Buckinghamshire MK18 3LA (GB)**
- **Fitzgerald, Mary.F.**
  **Yarntown,Oxfordshire OX5 1QB (GB)**
- **Sturton, Graham.**
  **Maidenhead.Berkshire. SL6 2DW (GB)**
- **NIEWÖHNER, Maria, Theresia**
  **42929 Wermelskirchen (DE)**

(56) References cited:
**EP-A- 0 009 384** **EP-A- 1 092 719**
**WO-A-01/64677** **WO-A-99/67244**
**DE-A- 2 811 780** **DE-A- 19 750 085**
**GB-A- 1 601 132** **GB-A- 1 601 133**
**US-A- 3 840 537** **US-A- 3 941 785**
**US-A- 5 932 578**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 399 439 B1

• **CHARLES I ET AL: "BICYCLIC HETEROCYCLES WITH NITROGEN AT THE RING JUNCTION. PART 2.1 APPLICATION OF THE DAKIN-WEST REACTION TO THE SYNTHESIS OF IMIDAZO-U5,1-F-1,2,4-TRIAZIN-4(3H)-ONES" JOURNAL OF THE CHEMICAL SOCIETY, PERKIN TRANSACTIONS 1, CHEMICAL SOCIETY. LETCHWORTH, GB, no. 5, 1 May 1980 (1980-05-01), pages 1139-1146, XP002027191 ISSN: 0300-922X**

Remarks:

The file contains technical information submitted after the application was filed and not included in this specification

## Description

The invention relates to 2-Heteroaryl-imidazotriazinones, processes for their preparation and their use in medicaments, esp. for the treatment and/or prophylaxis of inflammatory processes and/or immune diseases.

Phosphodiesterases (PDEs) are a family of enzymes responsible for the metabolism of the intracellular second messengers cAMP (cyclic adenosine monophosphate) and cGMP (cyclic guanosine monophosphate). PDE 4, as a cAMP specific PDE, catalyses the conversion of cAMP to AMP and is the major if not sole isoform of the phosphodiesterase enzymes present in inflammatory and immune cell types. Inhibition of this enzyme leads to the accumulation of cAMP which, in these cells, leads to the inhibition of a range of pro-inflammatory functions. Uncontrolled production of inflammatory mediators can lead to acute and chronic inflammation, tissue damage, multi-organ failures and to death. Additionally, elevation of phagocyte cAMP leads to inhibition of oxygen radical production. This cell function is more sensitive than others such as aggregation or enzyme release.

It is now recognised that both asthma and COPD (Chronic obstructive pulmonary disease) are chronic inflammatory lung diseases. In the case of asthma the eosinophil is the predominant infiltrating cell. Subsequent release of superoxide radicals as well as damaging cationic proteins from these infiltrating cells are believed to play a role in the progression of the disease and development of airway hyperreactivity.

By contrast, in COPD the neutrophil is the predominant inflammatory cell type found in the lungs of sufferers. The action of mediators and proteases released in the environment of the lung is believed to result in the irreversible airway obstruction seen in COPD. In particular the action of proteases in degrading the lung matrix results in fewer alveoli and is likely to be the major cause of accelerated long term lung function decline seen in this disease.

Treatment with a PDE 4 inhibitor is expected to reduce the inflammatory cell burden in the lung in both of these diseases [M.S. Barnette, "PDE 4 inhibitors in asthma and chronic obstructive pulmonary disease", in: Progress in Drug Research, Birkhäuser Verlag, Basel, 1999, pp. 193-229; H.J. Dyke and J.G. Montana, "The therapeutic potential of PDE 4 inhibitors", Exp. Opin. Invest. Drugs 8, 1301-1325 (1999)].

WO 99/24433 and WO 99/67244 describe 2-phenyl-imidazotriazinones as synthetic intermediates for the synthesis of 2-(aminosulfonyl-phenyl)-imidazotriazinones as inhibitors of cGMP-metabolizing phosphodiesterases.

US-A-4,278,673 discloses 2-aryl-imidazotriazinones with cAMP phosphodiesterase inhibitory activity for the treatment of i.a. asthma.

Further references may be found in the following documents: WO 01/64677, EP 1 092 719, DE 19 750 085, EP 0 009 384, US 3 941 785, DE 2 811 780, US 3 840 537, US 5 932 578, GB 1 601 132, GB 1 601 133 and Charles I et al. "Journal of the Chemical Society, Perkin Transactions 1, no 5, 1980 pages 1139-1146.

The present invention relates to compounds of the general formula (I)

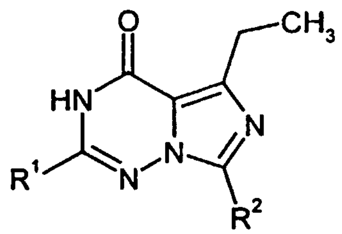

(I)

in which

$R^1$ denotes 5- to 10-membered heteroaryl, which is optionally substituted by identical or different residues selected from the group consisting of halogen, $(C_1-C_4)$-alkyl, trifluoromethyl, phenyl, cyano, nitro und trifluoromethoxy,

and

$R^2$ denotes 3- to 10-membered carbocyclyl or carbon-bonded, 4- to 10-membered heterocyclyl, whereby carbocyclyl and heterocyclyl are optionally substituted by identical or different residues selected from the group consisting of $(C_1-C_6)$-alkyl, $(C_1-C_6)$-alkoxy, hydroxy, halogen, trifluoromethyl and oxo, or denotes $(C_2-C_{10})$-alkyl, which is optionally substituted by identical or different residues selected from the group consisting of $(C_1-C_6)$-alkoxy, hydroxy, halogen, 3- to 10-membered carbocyclyl and oxo.

Another embodiment of the invention relates to compounds of the general formula (I), in which

$R^1$ denotes furanyl, thiophenyl, thiazolyl, pyridyl, chinolyl or isochinolyl, which are optionally substituted by identical

or different residues selected from the group consisting of halogen, $(C_1-C_4)$-alkyl, trifluoromethyl, cyano, nitro und trifluoromethoxy,

and $R^2$ has the meaning indicated above.

Another embodiment of the invention relates to compounds of the general formula (I), in which $R^1$ has the meaning indicated above, and

$R^2$ denotes $(C_4-C_7)$-cycloalkyl, which is optionally substituted up to two times by identical or different $(C_1-C_5)$-alkyl residues, or
denotes $(C_3-C_8)$-alkyl, which is optionally substituted by a $(C_4-C_7)$-cycloalkyl.

Preferred are compounds of the general formula (I), wherein $R^2$ denotes 4-*tert*-butylcyclohexyl.

Especially preferred are compounds of the general formula (I), wherein $R^2$ denotes *cis*-4-*tert*-butyl-cyclohexyl.

The compounds according to this invention can also be present in the form of their salts, hydrates and/or solvates.

In general, salts with organic or inorganic bases or acids may be mentioned here.

Physiologically acceptable salts are preferred in the context of the present invention.

Physiologically acceptable salts can also be salts of the compounds according to this invention with inorganic or organic acids. Preferred salts are those with inorganic acids such as, for example, hydrochloric acid, hydrobromic acid, phosphoric acid or sulphuric acid, or salts with organic carboxylic or sulphonic acids such as, for example, acetic acid, maleic acid, fumaric acid, malic acid, citric acid, tartaric acid, ethanesulphonic acid, benzenesulphonic acid, toluenesulphonic acid or naphthalenedisulphonic acid. Preferred pyridinium salts are salts in combination with halogen.

The compounds according to this invention can exist in stereoisomeric forms which either behave as image and mirror image (enantiomers), or which do not behave as image and mirror image (diastereomers). The invention relates both to the enantiomers and to the racemates, as well as the pure diastereomer and mixtures thereof. The racemates, like the diastereomers, can be separated into the stereoisomerically uniform constituents according to known methods.

Hydrates of the compounds of the invention are stoichiometric compositions of the compounds with water, such as for example hemi-, mono-, or dihydrates.

Solvates of the compounds of the invention or their salts are stoichiometric compositions of the compounds with solvents.

$(C_1-C_6)$-Alkoxy in general represents a straight chain or branched alkoxy residue with 1 to 6 carbon atoms. The following alkoxy residues are mentioned by way of example: methoxy, ethoxy, n-propoxy, isopropoxy, tert.butoxy, n-pentoxy and n-hexoxy. Alkoxy residues with 1 to 4 carbon atoms are preferred. Alkoxy residues with 1 to 3 carbon atoms are especially preferred.

$(C_2-C_{10})$-Alkyl, $(C_1-C_8)$-alkyl, $(C_1-C_6)$-alkyl, and $(C_1-C_4)$-alkyl in general represent straight chain or branched allcyl residues with 2 to 10, 1 to 8, 1 to 6 or 1 to 4 carbon atoms, respectively. The alkyl residues can be saturated or partially unsaturated, i.e. contain one or more double and/or triple bonds. Saturated alkyl residues are preferred. The following alkyl residues are mentioned by way of example: methyl, ethyl, n-propyl, isopropyl, allyl, propargyl, tert.butyl, pentyl, hexyl, heptyl, octyl, nonyl, and decyl.

$(C_6-C_{10})$-Aryl in general represents an aromatic residue with 6 to 10 carbon atoms. Phenyl and naphthyl are preferred.

3- to 10-membered carbocyclyl in general represents a mono- or polycyclic, carbocyclic residue with 3 to 10 ring atoms. 3- to 8-membered carbocyclyl is preferred. Mono- and bicyclic carbocyclyl residues are preferred. Especially preferred are monocyclic carbocyclyl residues. The carbocyclyl residues can be saturated or partially unsaturated. Saturated carbocyclyl residues are preferred. Especially preferred are $(C_3-C_{10})$-cycloalkyl and $(C_4-C_7)$-cycloalkyl residues. The following carbocyclyl residues are mentioned by way of example: cyclopropyl, cyclobutyl, cyclopentyl, cyclopentenyl, cyclohexyl, cycloheptyl, norborn-1-yl, norbom-2-yl, norbom-7-yl, norborn-2-en-7-yl, cyclooctyl, cubyl, cyclononyl, cyclodecyl, decalinyl, adamant-1-yl, adamant-2-yl.

$(C_3-C_{10})$-Cycloalkyl and $(C_4-C_7)$-cycloalkyl in general represent a cycloalkyl residue with 3 to 10 or 4 to 7 carbon atoms, respectively. The following cycloalkyl residues are mentioned by way of example: cyclopropyl, cyclobutyl, cyclopentyl, cyclopentenyl, cyclohexyl, cycloheptyl, cyclooctyl, cyclononyl, or cyclodecyl.

Halogen in general represents fluoro, chloro, bromo and iodo. Fluoro, chloro, and bromo are preferred. Fluoro, and chloro are especially preferred.

5- to 10-membered heteroaryl in general represents a mono- or bicyclic, heteroaromatic residue with 5 to 10 ring atoms. Up to 4, preferably up to 2 ring atoms can be identical or different heteroatoms, preferably selected from N, O, and S. The following heteroaryl residues are mentioned by way of example: thienyl, furyl, pyrrolyl, imidazolyl, pyrazolyl, triazolyl, tetrazolyl, oxazolyl, thiazolyl, isothiazolyl, pyridyl, pyrimidyl, pyrazinyl, pyridazinyl, indolyl, quinolyl, isoquinolyl, quinazolyl, quinoxazolyl, benzothiazolyl, benzisothiazolyl, benzoxazolyl, isoxazolyl, benzimidazolyl, and oxazolinyl.

Carbon-bonded, 4- to 10-membered heterocyclyl in general represents a mono- or polycyclic, heterocyclic residue with 4 to 10 ring atoms, whereby the heterocycle is bound through a ring carbon ring atom. The heterocyclyl residue can contain up to 3, preferably 1, hetero ring atoms selected from nitrogen, oxygen, sulfur, -SO-, $-SO_2-$. Oxygen is preferred. Mono- and bicyclic heterocyclyl residues are preferred. Especially preferred are monocyclic heterocyclyl residues. The heterocyclyl residues can be saturated or partially unsaturated. Saturated heterocyclyl residues are preferred. The following heterocyclyl residues are mentioned by way of example: oxetan-3-yl, pyrrolidin-2-yl, pyrrolidin-3-yl, pyrrolinyl, tetrahydrofuranyl, tetrahydrothienyl, pyranyl, piperidinyl, thiopyranyl, morpholinyl, perhydroazepinyl.

Oxo in general represents a double-bonded oxygen atom.

Unless specified otherwise, when groups in compounds of the invention are optionally substituted, substitution by up to three identical or different residues is generally preferred.

The invention furthermore provides a process for preparing the compounds of the general formula (I) according to the invention, characterized in that compounds of the general formula (II)

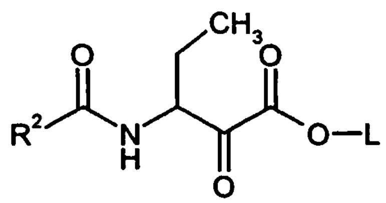

**(II)**

in which

$R^2$ is as defined above

and

L represents straight-chain or branched alkyl having up to 4 carbon atoms,

are condensed with compounds of the general formula (III)

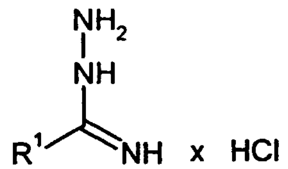

**(III)**

in which

$R^1$ is as defined above,

preferably using ethanol as a solvent, to the compounds of the general formula (IV),

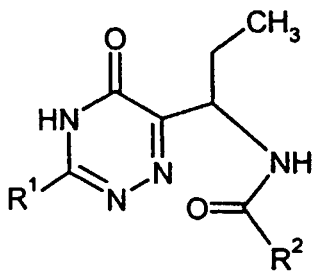

**(IV)**

in which $R^1$ and $R^2$ are as defined above,

which can optionally after isolation be reacted with a dehydrating agent, preferably phosphorus oxytrichloride, to yield the compounds of the general formula (I).

The compounds of the general formula (IV) can alternatively be prepared by

[A] condensation of compounds of the general formula (IIa),

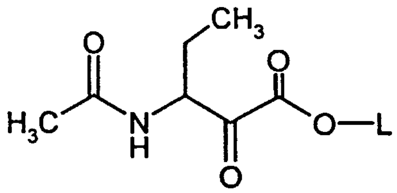

(IIa)

in which

L is as defined above,

with compounds of the general formula (III) to compounds of the general formula (IVa),

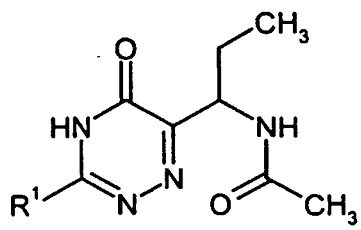

(IVa)

in which

$R^1$ is as defined above,

preferably using ethanol as a solvent,

[B] followed by hydrolysis of the compounds of the general formula (IVa) to compounds of the general formula (V),

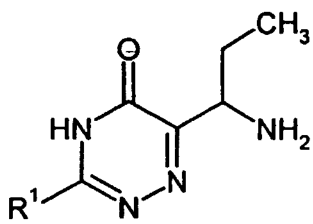

(V)

in which

$R^1$ is as defined above,

[C] and finally by condensation of the compounds of the general formula (V) with compounds of the general formula (VI),

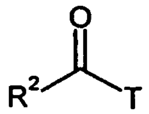

(VI)

in which

$R^2$ is as defined above, and

T represents a leaving group, preferably chlorine.

The process according to the invention can be illustrated using the following scheme as an example:

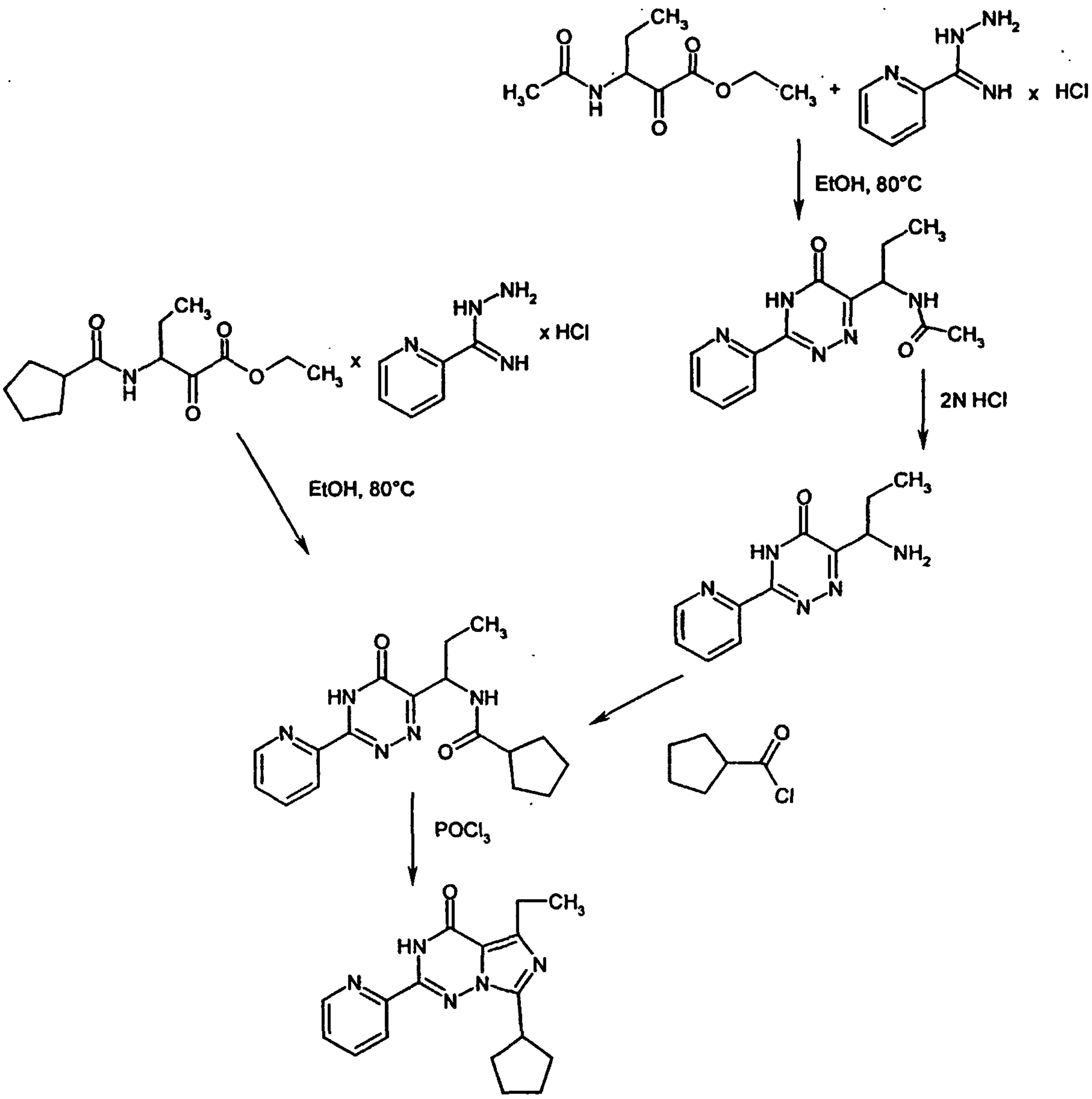


Solvents which are suitable for the individual steps are the customary organic solvents which do not change under the reaction conditions. These preferably include ethers, such as diethyl ether, dioxane, tetrahydrofuran, glycol dimethyl ether, or hydrocarbons, such as benzene, toluene, xylene, hexane, cyclohexane or mineral oil fractions, or halogenated hydrocarbons, such as dichloromethane, trichloromethane, carbon tetrachloride, dichloroethane, trichloroethylene or chlorobenzene, or ethyl acetate, dimethylformamide, hexamethylphosphoric triamide, acetonitrile, acetone, dimethoxyethane or pyridine. It is also possible to use mixtures of the abovementioned solvents. Particular preference is given to ethanol for the reaction II/IIa + III → IV/IVa and dichloroethane for the cyclisation IV → I.

The reaction temperature can generally be varied within a relatively wide range. In general, the reaction is carried out in a range of from -20°C to 200°C, preferably of from 0°C to 100°C.

The process steps according to the invention are generally carried out under atmospheric pressure. However, it is also possible to operate under superatmospheric pressure or under reduced pressure (for example, in a range of from 0.5 to 5 bar).

The compounds of the general formula (IVa) are preferably hydrolysed to compounds of the general formula (V) under acidic conditions as for example in refluxing 2N hydrochloric acid.

The compounds of the general formula (V) are condensed with the compounds of the general formula (VI) to compounds of the general formula (IV) in inert solvents, if appropriate in the presence of a base.

Suitable inert solvents are the customary organic solvents which do not change under the reaction conditions. These preferably include ethers, such as diethyl ether, dioxane, tetrahydrofuran, glycol dimethyl ether, or hydrocarbons, such as benzene, toluene, xylene, hexane, cyclohexane or mineral oil fractions, or halogenated hydrocarbons, such as dichloromethane, trichloromethane, carbon tetrachloride, dichloroethylene, trichloroethylene or chlorobenzene, or ethyl acetate, dimethylformamide, hexamethylphosphoric triamide, acetonitrile, acetone, dimethoxyethane or pyridine. It is also possible to use mixtures of the abovementioned solvents.

Suitable bases are generally alkali metal hydrides or alkali metal alkoxides, such as, for example, sodium hydride or potassium tert-butoxide, or cyclic amines, such as, for example, piperidine, pyridine, dimethylaminopyridine or ($C_1$-$C_4$)- alkylamines, such as, for example, triethylamine. Preference is given to triethylamine, pyridine and/or dimethylaminopyridine.

The base is generally employed in an amount of from 1 mol to 4 mol, preferably from 1.2 mol to 3 mol, in each case based on 1 mol of the compound of the formula (V).

The reaction temperature can generally be varied within a relatively wide range. In general, the reaction is carried out in a range of from -20°C to 200°C, preferably of from 0°C to 100°C.

Some of the compounds of the general formula (II) are known, or they are novel, and they can then be prepared by

converting compounds of the general formula (VI)

$R^2$-CO-T (VI)

in which

$R^2$ is as defined above

and

T represents halogen, preferably chlorine,

initially by reaction with α-amino-butyric acid in inert solvents, if appropriate in the presence of a base and trimethylsilyl chloride, into the compounds of the general formula (VII),

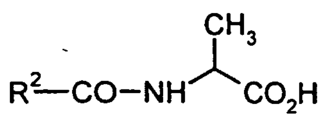

(VII)

in which

$R^2$ is as defined above,

and finally reacting with the compound of the formula (VIII)

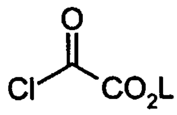

(VIII)

in which

L is as defined above,

in inert solvents, if appropriate in the presence of a base.

The compounds of the general formula (IIa) can be prepared analogously.

Suitable solvents for the individual steps of the process are the customary organic solvents which do not change under the reaction conditions. These preferably include ethers, such as diethyl ether, dioxane, tetrahydrofuran, glycol dimethyl ether, or hydrocarbons, such as benzene, toluene, xylene, hexane, cyclohexane or mineral oil fractions, or halogenated hydrocarbons, such as dichloromethane, trichloromethane, carbon tetrachloride, dichloroethylene, trichloroethylene or chlorobenzene, or ethyl acetate, dimethylformamide, hexamethylphosphoric triamide, acetonitrile, acetone, dimethoxyethane or pyridine. It is also possible to use mixtures of the abovementioned solvents. Particular preference is given to dichloromethane for the first step and to a mixture of tetrahydrofuran and pyridine for the second step.

Suitable bases are generally alkali metal hydrides or alkali metal alkoxides, such as, for example, sodium hydride or potassium tert-butoxide, or cyclic amines, such as, for example, piperidine, pyridine, dimethylaminopyridine or ($C_1$-$C_4$)-alkylamines, such as, for example, triethylamine. Preference is given to triethylamine, pyridine and/or dimethylaminopyridine.

The base is generally employed in an amount of from 1 mol to 4 mol, preferably from 1.2 mol to 3 mol, in each case based on 1 mol of the compound of the formula (X).

The reaction temperature can generally be varied within a relatively wide range. In general, the reaction is carried out in a range of from -20°C to 200°C, preferably of from 0°C to 100°C.

The compounds of the general formulae (VI) and (VIII) are known per se, or they can be prepared by customary methods.

The compounds of the general formula (III) are known or can be prepared by reacting compounds of the general formula (IX)

$R^1$-Y (IX)

in which

$R^1$ is as defined above, and

Y represents a cyano, carboxyl, methoxycarbonyl or ethoxycarbonyl group,

with ammonium chloride in toluene and in the presence of trimethylaluminium in hexane in a temperature range of from -20°C to room temperature, preferably at 0°C and atmospheric pressure, and reacting the resulting amidine, if appropriate in situ, with hydrazine hydrate.

The compounds of the general formula (IX) are known per se, or they can be prepared by customary methods.

The compounds of the general formula (I) inhibit the PDE 4 resident in the membranes of human neutrophils. One measured functional consequence of this inhibition was inhibition of superoxide anion production by stimulated human neutrophils.

The compounds of the general formula (I) can therefore be employed in medicaments for the treatment of inflammatory processes, esp. acute and chronic inflammatory processes, and/or immune diseases.

The compounds according to the invention are preferably suitable for the treatment and prevention of inflammatory processes, i.e. acute and chronic inflammatory processes, and/or immune diseases, such as emphysema, alveolitis, shock lung, all kinds of chronic obstructive pulmonary diseases (COPD), adult respiratory distress syndrome (ARDS), asthma, bronchitis, cystic fibrosis, eosinophilic granuloma, arteriosclerosis, arthrosis, inflammation of the gastrointestinal tract, myocarditis, bone resorption diseases, reperfusion injury, Crohn's disease, ulcerative colitis, systemic lupus erythematosus, type I diabetes mellitus, psoriasis, anaphylactoid purpura nephritis, chronic glomerulonephritis, inflammatory bowel disease, atopic dermatitis, other benign and malignant proliferative skin diseases, allergic rhinitis, allergic conjunctivitis, vemal conjunctivitis, arterial restenosis, sepsis and septic shock, toxic shock syndrome, grafts vs.

host reaction, allograft rejection, treatment of cytokine-mediated chronic tissue degeneration, rheumatoid arthritis, arthritis, rheumatoid spondylitis, osteoarthritis, coronary insufficiency, myalgias, multiple sclerosis, malaria, AIDS, cachexia, prevention of tumor growth and tissue invasion, leukemia, depression, memory impairment and acute stroke. The compounds according to the invention are additionally suitable for reducing the damage to infarct tissue after reoxygenation.

The compounds of formula (I) according to the invention can be used as active compound components for the production of medicaments. For this, they can be converted into the customary formulations such as tablets, coated tablets, aerosols, pills, granules, syrups, emulsions, suspensions and solutions in a known manner using inert, non-toxic, pharmaceutically suitable excipients or solvents. Preferably, the compounds according to the invention are used here in an amount such that their concentration in the total mixture is approximately 0.5 to approximately 90% by weight, the concentration, inter alia, being dependent on the corresponding indication of the medicament.

The above mentioned formulations are produced, for example, by extending the active compounds with solvents and/or excipients having the above properties, where, if appropriate, additionally emulsifiers or dispersants and, in the case of water as the solvent, alternatively an organic solvent, have to be added.

Administration is carried out in a customary manner, preferably orally, transdermally or parenterally, for example perlingually, buccally, intravenously, nasally, rectally or inhalationally.

For human use, in the case of oral administration, it is recommendable to administer doses of from 0.001 to 50 mg/kg, preferably of 0.01 mg/kg - 20 mg/kg. In the case of parenteral administration, such as, for example, intravenously or via mucous membranes nasally, buccally or inhalationally, it is recommendable to use doses of 0.001 mg/kg - 0.5 mg/kg.

In spite of this, if appropriate, it may be necessary to depart from the amounts mentioned above, namely depending on the body weight or the type of administration route, on the individual response towards the medicament, the manner of its formulation and the time or interval at which administration takes place. Thus, in some cases it may be sufficient to manage with less than the above mentioned minimum amount, while in other cases the upper limit mentioned must be exceeded. In the case of the administration of relatively large amounts, it may be recommendable to divide these into several individual doses over the course of the day.

Test descriptions

1. Preparation of human PMN
Human PMN (polymorphonuclear neutrophil leucocytes) are readily purified from peripheral blood. Phosphodiesterase in these cells is predominantly located in the membrane fraction. Inhibitory potency of compounds against this preparation correlate well with the anti-inflammatory activity as measured by inhibiton of superoxide production.
Blood was taken from healthy subjects by venous puncture and neutrophils were purified by dextran sedimentation and density gradient centrifugation on Ficoll Histopaque and resuspended in the buffered medium.

2. Assay of human PMN phosphodiesterase
This was performed as a particulate fraction from human PMN essentially as described by Souness and Scott [Biochem. J. 291, 389-395 (1993)]. Particulate fractions were treated with sodium vanadate / glutathione as described by the authors to express the discrete stereospecific site on the phosphodiesterase enzyme. The prototypical PDE 4 inhibitor, rolipram, had an $IC_{50}$ value in the range 450 nM-1500 nM, thus defining this preparation as the so-called "low affinity" [L] form. The preparation examples had $IC_{50}$ values within the range of 0.1 nM - 10,000 nM.

3. Inhibition of FMLP-stimulated production of superoxide radical anions
Neutrophils ($2.5 \times 10^5$ $Ml^{-1}$) were mixed with cytochrome C (1.2 mg/ml) in the wells of a microtitre plate. Compounds according to the invention were added in dimethyl sulphoxide (DMSO). Compound concentration ranged from 2.5 nM to 10 $\mu$M, the DMSO concentration was 0.1% v/v in all wells. After addition of cytochalasin b (5 $\mu g \times ml^{-1}$) the plate was incubated for 5 min at 3.7°C. Neutrophils were then stimulated by addition of $4 \times 10^{-8}$ M FMLP (N-Formyl-Met-Leu-Phe) and superoxide generation measured as superoxide dismutase inhibitable reduction of cytochrome C by monitoring the $OD_{550}$ in a Thermomax microtitre plate spectrophotometer. Initial rates were calculated using a Softmax kinetic calculation programme. Blank wells contained 200 units of superoxide dismutase.
The inhibition of superoxide production was calculated as follows:

$$\frac{[1-(Rx - Rb)]}{(Ro - Rb)} \times 100$$

Rx = Rate of the well containing the compound according to the invention
Ro = Rate in the control well
Rb = Rate in the superoxide dismutase containing blank well

4. Assay of binding to the rolipram binding site (PDE 4 high affinity site; "H-PDE 4 form") in rat brain membranes
The activity of compounds on the PDE 4 high affinity site ("H-PDE 4 form") is readily measured by determining their potency for displacement of [$^3$H]-rolipram from its binding site in rat brain membranes. Activity at this site is believed to be a measure of side effect potential (e.g. stimulation of stomach acid secretion, nausea and emesis).
The rolipram binding site assay was performed essentially as described by Schneider et al. [Eur. J. Pharmacol. 127, 105-115 (1986)].

5. Lipopolysaccharide (LPS) - induced neutrophil influx into rat lung Intranasal administration of LPS to rats causes a marked influx of neutrophils into the lungs measurable by histological or biochemical (myeloperoxidase content of the cell pellet) analysis of the bronchoalveolar lavage fluid 24 h later. Rats were treated with test compound or vehicle administered by the oral route 1 h prior to and 6 h after administration of intranasal LPS. 24 hours later animals were euthanatized and their lungs lavaged with PBS (phosphate buffered saline). Neutrophil and total cell numbers were analysed.

6. Emetic potential in the marmoset
Vehicle or test compound was administered by the oral route to conscious marmosets. Animals were observed for emetic episodes or abnormal behaviour for 1 h post dosing. In some experiments, if no adverse response was seen, a separate group of animals was tested at ½ log dose higher until emesis or abnormal behaviour was observed. The highest dose at which no abnormal behavior or emetic episodes occurred was recorded as the NOEL.

**Materials and Methods**

**LC-MS method A**

| | | | | |
|---|---|---|---|---|
| LC-parameters | solution A acetonitrile | | | |
| | solution B 0.3 g 30% HCl/l water | | | |
| | column oven 50°C; | | | |
| | column Symmetry C18 2.1 x 150 mm | | | |
| gradient: | time [min] | %A | %B | flow [ml/min] |
| | 0 | 10 | 90 | 0.9 |
| | 3 | 90 | 10 | 1.2 |
| | 6 | 90 | 10 | 1.2 |

**LC-MS method B**

| | |
|---|---|
| LC-parameters | solution A acetonitrile/0.1% formic acid |
| | solution B water/0.1% formic acid |
| | column oven 40°C; |
| | column Symmetry C18 2.1 x 50 mm |

Table continued

| gradient : | time [min] | %A | %B | flow [ml/min] |
|---|---|---|---|---|
| | 0 | 10 | 90 | 0.5 |
| | 4 | 90 | 10 | 0.5 |
| | 6 | 90 | 10 | 0.5 |
| | 6.1 | 10 | 90 | 1.0 |
| | 7.5 | 10 | 90 | 0.5 |

**GC-MS method A**

| | |
|---|---|
| Column: | HP-5 30m x 320μm x 0.25μm |
| Carrier Gas: | Helium |
| Mode: | constant flow, initial flow: 1.5 ml/min |
| Oven ramp: | initial temp: 60°C |
| | initial time: 1min |
| | rate: 14°C/min up to 300°C, then 300°C 2 min |

Unless specified otherwise, the following chromatographic conditions were applied: chromatography was performed on silica gel Si 60; for flash chromatography, the usual conditions were followed as described in Still, *J. Org. Chem.* 43, 2923 (1978); mixtures of dichloromethane and methanol or cyclohexane and ethylacetate were used as eluants. Unless specified otherwise, reactions were executed under an argon atmosphere and under anhydrous conditions.

**Abbreviations**

| | |
|---|---|
| HPLC = | high performance liquid chromatography |
| MS = | mass spectroscopy |
| NMR = | nuclear magnetic resonance spectroscopy |
| LC-MS = | liquid chromatography combined with mass spectroscopy |
| GC-MS = | gas chromatography combined with mass spectroscopy |
| MeOH = | methanol |
| DMSO = | dimethylsulfoxide |

**Starting Materials**

**Example 1A**

2-(Acetylamino)butanoic acid

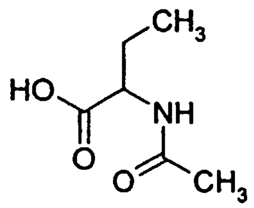

163 g (1.58 mol) 2-Aminobutanoic acid are dissolved in acetic acid, and 242 g (2.37 mol) acetic anhydride are added dropwise. The mixture is stirred for 2 h at 100°C until completion of reaction, then the solution evaporated to dryness *in vacuo*. The solid residue is suspended in ethyl acetate, filtered and washed with diethyl ether.
Yield: 220 g (96%)
$^1$H-NMR (Methanol-$d_4$): δ = 0,97 (t, 3 H), 1,65-1,93 (m, 2 H), 1,99 (s, 3 H), 4,29 (q, 1 H) ppm.

**Example 2A**

Ethyl 3-(acetylamino)-2-oxopentanoate

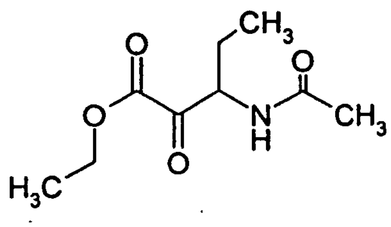

9.2 g (63.4 mmol) 2-(Acetylamino)butanoic acid are suspended in 120 ml tetrahydrofurane and heated to reflux together with 15.0 g (190 mmol) pyridine and a bit of *N,N*-dimethylaminopyridine. While heating at reflux, 17.3 g (127 mmol) ethyl chloro(oxo)acetate are added dropwise. The reaction mixture is heated at reflux until no more evolution of gas can be observed. After cooling down to room temperature, the reaction mixture is added to ice water and the organic phase extracted with ethyl acetate. The dried organic phase is evaporated to dryness *in vacuo*, dissolved in ethanol and the solution directly used for the next reaction.

**Example 3A**

2-[(Cyclopentylcarbonyl)amino]butanoic acid

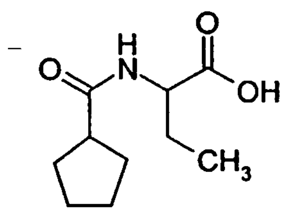

35 g (339 mmol) 2-aminobutanoic acid and 75,6 g (747 mmol) triethylamine are suspended in 300 ml of dichloromethane and stirred at 0°C. 81 g(747 mmol) chlorotrimethylsilane are added dropwise, then the mixture is stirred for 1hour at room temperature and 1hour at 40°C. After cooling down at -10°C, 45 g (339 mmol) cyclopentanecarbonyl chloride are added slowly. The reaction mixture is stirred for 2 hours at -10°C and then 1 hour at room temperature. At 0°C, 50 ml of water are added. The mixture is diluted with water and dichloromethane, filtered and the solid product

washed with water/dichloromethane 9/1, toluene and diethylether.
Yield: 52.4 g (77%)
$^1$H-NMR (DMSO-$d_6$, 300 MHz): δ = 0,9 (t, 3H), 1,6 (m, 10H), 2,6 (m, 1H), 4,1 (m, 2H), 7,9 (d, 1H), 12,4 (s, 1H) ppm.

**Example 4A**

Ethyl 3-[(cyclopentylcarbonyl)amino]-2-oxopentanoate

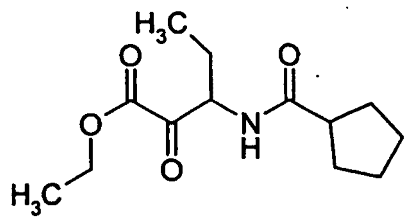

1,6 g (8 mmol) 2-[(Cyclopentylcarbonyl)amino]butanoic acid are suspended in 30 ml tetrahydrofurane and heated to reflux together with 1,91 g (24 mmol) pyridine and a bit of *N,N*-dimethylaminopyridine. While heating at reflux, 2,19 g (16 mmol) ethyl chloro(oxo)acetate are added dropwise. The reaction mixture is heated at reflux until no more evolution of gas can be observed.. After cooling down to room temperature, the reaction mixture is added to ice water and the organic phase extracted with ethyl acetate. The dried organic phase is evaporated to dryness *in vacuo*, dissolved in ethanol and the solution directly used for the next reaction.

**Example 5A**

3-Thiophenecarboximidamide hydrochloride

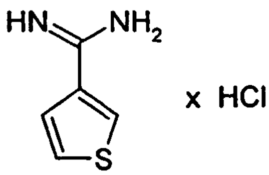

5,91 g (91,6 mmol, 2 equiv.) ammonium chloride are suspended in 40 ml of dry toluene under an argon atmosphere, and the mixture is cooled to 0°C. 45,8 ml (91,6 mmol, 2 equiv.) of a 2M solution of trimethylaluminium in hexane are added dropwise, and the reaction mixture is stirred at room temperature until no more evolution of gas is observed. After addition of 5,0 g (45,8 mmol) thiophene-3-carbonitrile, the mixture is stirred at 80°C bath temperature over night. It is then cooled down to 0°C and 50 ml of methanol are added with consequent stirring of 1 hour at room temperature. After filtration, the solid is washed with methanol for several times, the solution is evaporated to dryness in vacuo and the residue washed with methanol.
Yield: 6.7 g (90%)
$^1$H-NMR (DMSO-$d_6$, 200 MHz): δ = 7,7 (m, 1H), 7,8 (m, 1H), 8,7 (m, 1H), 9,0 (br.s, 2H), 9,4 (br.s, 2H) ppm.

**Example 6A**

2-Quinolinecarboximidamide hydrochloride

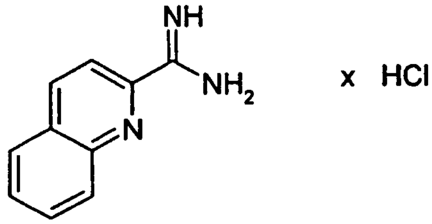

In analogy to the procedure for Example 5A, 10,0 g (64,9 mmol) 2-quinoline-carbonitrile and proportionate amounts of the other reagents are used.
Yield: 9.2 g (68%)
$^1$H-NMR (200 MHz, DMSO): δ = 7,83 (t, 1 H), 7,97 (t, 1 H), 8,19 (t, 2 H), 8,37 (d, 1 H), 8,77 (d, 1 H) ppm.

**Example 7A**

N-{1-[5-Oxo-3-(3-thienyl)-4,5-dihydro-1,2,4-triazin-6-yl]propyl}acetamide

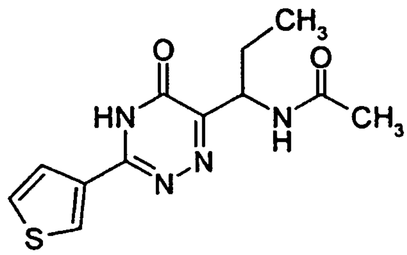

6,5 g (8,6 mmol, 1 equiv.) (40 mmol) of Example 5A are suspended in 150 ml of ethanol and 6,92 g (48 mmol, 1,2 equiv.) hydrazine hydrate are added. After stirring at room temperature for 1 hour, 11,95 g (60 mmol, 1,5 equiv) of the compound of Example 2A, dissolved in 30 ml of ethanol, are added. The reaction mixture is stirred at 80°C (bath temperature) for 4 hours and then at room temperature over night. The mixture is evaporated to dryness *in vacuo* and the product is purified by chromatography (flash or column chromatography or preparative HPLC).
Yield: 4.9 g (44%)
$^1$H-NMR (DMSO-$d_6$, 300 MHz): δ = 0,9 (t, 3H), 1,6 (m, 1H), 1,8 (m, 1H), 1,9 (s, 3H), 4,9 (m, 1H), 7,7 (m, 2H), 8,1 (m, 1H), 8,5 (m, 1H), 14,0 (br. s, 1H) ppm.

**Example 8A**

N-{1-[5-Oxo-3-(2-phenyl-1,3-thiazol-4-yl)-4,5-dihydro-1,2,4-triazin-6-yl]propyl}-acetamide

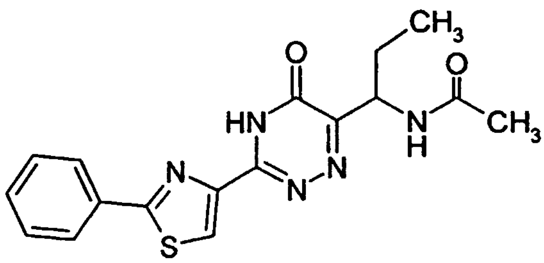

In analogy to the procedure for Example 7A, 1,0 g (4,2 mmol) 2-phenyl-1,3-thiazole-4-carboximidamide hydrochloride and proportionate amounts of the other reagents are used.
Yield: 655 mg (44%)
$^1$H-NMR (DMSO-$d_6$, 200 MHz): $\delta$ = 0,9 (t, 3H), 1,6 (m, 1H), 1,8 (m, 1H), 1,9 (s, 3H), 4,9 (m, 1H), 7,6 (m, 3H), 8,2 (m, 2H), 8,7 (s, 1H), 14,2 (br. s, 1H) ppm.

**Example 9A**

N-{1-[5-Oxo-3-(2-quinolinyl)-4,5-dihydro-1,2,4-triazin-6-yl]propyl}acetamide

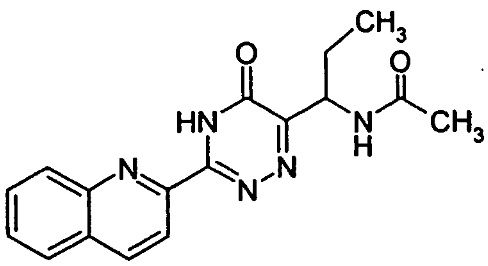

In analogy to the procedure for Example 7A, 5,0 g (24,1 mmol) 2-quinolinecarboximidamide hydrochloride and proportionate amounts of the other reagents are used.
Yield: 6.0 g (54%)
LC/MS (method A): retention time 2.05 min., *m/z* 324 $[M+H]^+$

**Example 10A**

N-{1-[5-Oxo-3-(2-thienyl)-4,5-dihydro-1,2,4-triazin-6-yl]propyl}acetamide

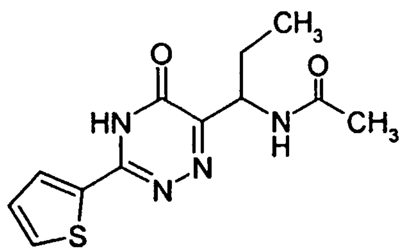

In analogy to the procedure for Example 7A, 2,0 g (12,3 mmol) 2-thiophenecarboximidamide hydrochloride and proportionate amounts of the other reagents are used.
Yield: 0.6 g (15%)
$^1$H-NMR (DMSO-$d_6$, 200 MHz): $\delta$ = 0,9 (t, 3H), 1,6 (m, 1H), 1,8 (m, 1H), 1,9 (s, 3H), 4,9 (m, 1H), 7,3 (m, 1H), 8,0- 8,2 (m, 3H), 14,2 (br. s, 1H) ppm.

**Example 11A**

6-(1-Aminopropyl)-3-(3-thienyl)-1,2,4-triazin-5(4H)-one

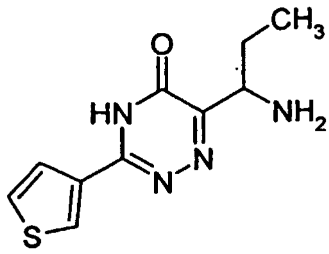

4,9 g (17,6 mmol) Example 7A are heated to reflux in 50 ml 2 N hydrochloric acid for 3 hours. After cooling down to room teperature, the mixture is neutralized with 10% NaOH and, and, after addition of ethanol, evaporated to dryness *in vacuo.* The residue is treated with methanol and the filtrate separated from th salts. The filtrate is evaporated to dryness in vacuo and the crude product is directly used for the next step or the product is purified by chromatography (flash or column chromatography or preparative HPLC).
crude product:
LC/MS (B): MS (ES+): 237 ($M+H^+$), retention time 0.38 min

**Example 12A**

6-(1-Aminopropyl)-3-(2-phenyl-1,3-thiazol-4-yl)-1,2,4-triazin-5(4H)-one

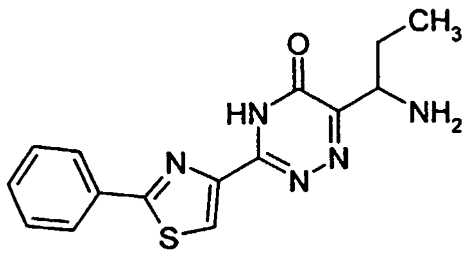

In analogy to the procedure for Example 11A, 631 mg (1,8 mmol) of Example 8A and proportionate amounts of the other reagents are used.
Yield: 373 mg (67%)
$^1$H-NMR (DMSO-$d_6$, 200 MHz): $\delta$ = 1,2 (t, 3H), 1,7 (m, 1H), 1,9 (m, 1H), 3,9 (d/d, 1H), 4,9 (br.s, 2H), 7,5 (m, 3H), 8,0 (m, 2H), 8,2 (s, 1H) ppm.

**Example 13A**

6-(1-Aminopropyl)-3-(2-quinolinyl)-1,2,4-triazin-5(4H)-one

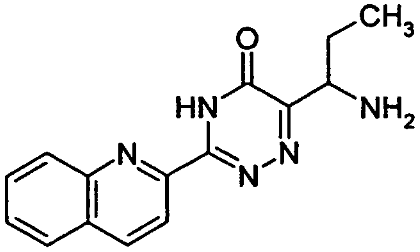

In analogy to the procedure for Example 11A, 6,0 g (18,6 mmol) N-{1-[5-oxo-3-(2-quinolinyl)-4,5-dihydro-1,2,4-triazin-6-yl]propyl}acetamide and proportionate amounts of the other reagents are used.
Yield: 3.1 g (42%)
MS (ESI+): 282 $[M+H]^+$

**Example 14A**

6-(1-Aminopropyl)-3-(2-thienyl)-1,2,4-triazin-5(4H)-one

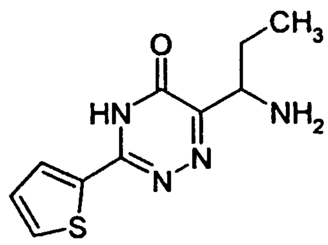

In analogy to the procedure for Example 11A, 9,40 g (33,8 mmol) of Example 10A and proportionate amounts of the other reagents are used.
Yield: 5.07 g (63%)
$^1$H-NMR (DMSO-$d_6$, 200 MHz): $\delta$ = 0,9 (t, 3H), 1,9 (m, 2H), 4,2 (bs, 1H), 4,3 (m, 1H), 7,1 (dd, 1H), 7,7 (m, 1H), 7,8 (m, 1H), 8,3 (br. s, 2H) ppm.

**Example 15A**

N-{1-[5-Oxo-3-(2-phenyl-1,3-thiazol-4-yl)-4,5-dihydro-1,2,4-triazin-6-yl]propyl}-cyclopentanecarboxamide

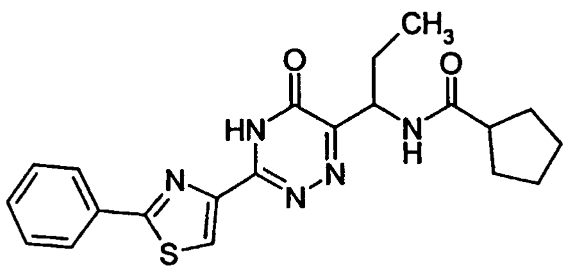

170 mg (0,54 mmol, 1 equiv.) of Example 12A are suspended in 10 ml dichloromethane, 0,15 ml (1,08 mmol, 2 equiv.) triethylamine and 0,066 ml (0,54 mmol, 1 equiv.) cyclopentanecarbonyl chloride are added. The reaction mixture is stirred at room temperature until completion of reaction (1-2 hours). The reaction mixture is added to the same volume of 1N hydrochloric acid, the organic phase is washed with 1N hydrochloric acid and brine, dried over sodium sulfate and evaporated to dryness. The product is used without further purification or purified by chromatography (flash or column chromatography or preparative HPLC).
Yield: 182 mg (82%)
$^1$H-NMR (DMSO-$d_6$, 200 MHz): $\delta$ = 1,2 (t, 3H), 1,6-1,9 (m, 10H), 2,6 (m, 1H), 4,9 (m, 1H), 7,6 (m, 3H), 8,0 (d, 1H), 8,2 (m, 2H), 8,7 (s, 1H), 14,2 (br. s, 1H) ppm.

**Example 16A**

N-{1-[5-Oxo-3-(2-phenyl-1,3-thiazol-4-yl)-4,5-dihydro-1,2,4-triazin-6-yl]propyl}-cyclobutanecarboxamide

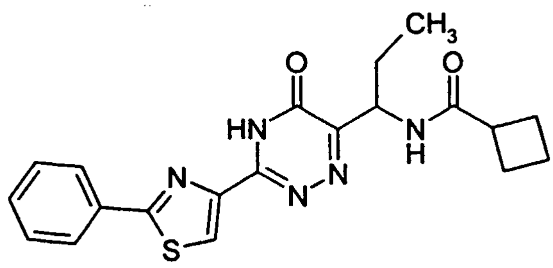

In analogy to the procedure for Example 15A, 188 mg (0,6 mmol) of Example 12A, 0,068 ml (0,6 mmol) cyclobutanecarbonyl chloride and proportionate amounts of the other reagents are used.
Yield: 218 mg (92%)
$^1$H-NMR (DMSO-$d_6$, 200 MHz): δ = 1,2 (t, 3H), 1,6-2,1 (m, 8H), 3,1 (m, 1H), 4,9 (m, 1H), 7,6 (m, 3H), 7,9 (d, 1H), 8,2 (m, 2H), 8,7 (s, 1H), 14,2 (br. S(1H) ppm.

**Example 17A**

N-{1-[5-Oxo-3-(3-thienyl)-4,5-dihydro-1,2,4-triazin-6-yl]propyl}cyclopentanecarboxamide

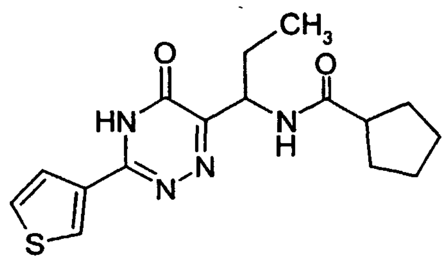

In analogy to the procedure for Example 15A, 400 mg (1,69 mmol) of Example 11A, 0,206 ml (1,69 mmol) cyclopentanecarbonyl chloride and proportionate amounts of the other reagents are used.
LC/MS (B): MS (ES+): 333 (M+H$^+$), retention time 3.05 min.

**Example 18A**

N-{1-[5-Oxo-3-(3-thienyl)-4,5-dihydro-1,2,4-triazin-6-yl]propyl}cyclobutanecarboxamide

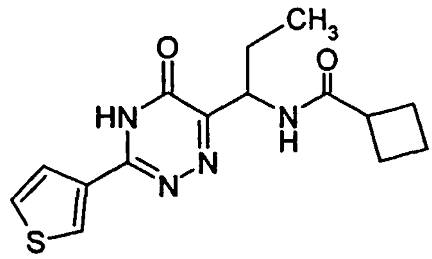

In analogy to the procedure for Example 15A, 400 mg (1,69 mmol) of Example 11A, 0,193 ml (1,69 mmol) cyclobutanecarbonyl chloride and proportionate amounts of the other reagents are used.
LC/MS (B): MS (ES+): 319 ($M+H^+$), retention time 2.82 min.

**Example 19A**

4-tert-Butyl-N-{1-[5-oxo-3-(3-thienyl)-4,5-dihydro-1,2,4-triazin-6-yl]propyl}cyclohexanecarboxamide

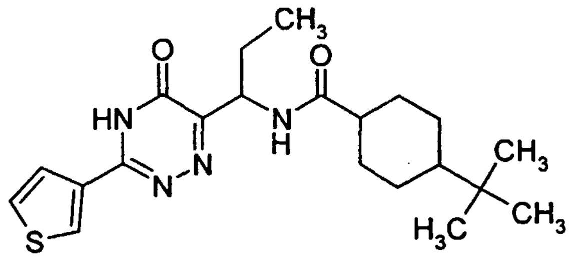

In analogy to the procedure for Example 15A, 400 mg (1,69 mmol) of Example 11A, 343 mg (1,69 mmol) 4-tert-butylcyclohexanecarbonyl chloride and proportionate amounts of the other reagents are used.
LC/MS (B): MS (ES+): 403 ($M+H^+$), retention time 4.16 min.

**Example 20A**

N-{1-[5-Oxo-3-(2-quinolinyl)-4,5-dihydro-1,2,4-triaziri-6-yl]propyl}cyclopentanecarboxamide

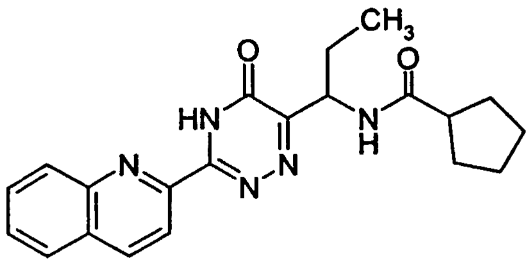

In analogy to the procedure for Example 15A, 500 mg (1,78 mmol) 6-(1-aminopropyl)-3-(2-quinolinyl)-1,2,4-triazin-5(4H)-one, 350 mg (2,67 mmol) cyclopentanecarbonyl chloride and proportionate amounts of the other reagents are used. The crude product is used in the next step without further purification.

Yield: 275 mg (41%) crude product.

**Example 21A**

N-{1-[5-Oxo-3-(4-pyridinyl)-4,5-dihydro-1,2,4-triazin-6-yl]propyl}cyclopentanecarboxamide

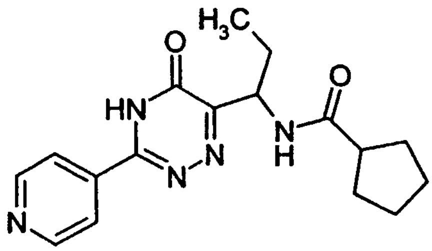

560 mg (3,56 mmol, 1 equiv.) 4-pyridinecarboximidamide hydrochloride are suspended in 10 ml of ethanol and 220 mg (4,27 mmol, 1,2 equiv.) hydrazine hydrate are added. After stirring at room temperature for 1 hour, 1,0 g (3,92 mmol, 1,1 equiv) of the compound of Example 4A, dissolved in 10 ml of ethanol, are added. The reaction mixture is stirred at 70°C (bath temperature) for 4 hours. The mixture is evaporated to dryness *in vacuo* and the product is purified by chromatography (flash or column chromatography or preparative HPLC).
Yield: 400 mg (34%)
$^1$H-NMR (DMSO-$d_6$, 200 MHz): δ = 0,9 (t, 3H), 1,4-1,9 (m, 10H), 2,7 (m, 1H), 4,9 (m, 1H), 8,0 (m, 3H), 8,8 (d, 2H), 14,3 (br. s, 1H) ppm.

**Example 22A**

N-{1-[3-(4,6-Dimethyl-2-pyridinyl)-5-oxo-4,5-dillydro-1,2,4-triazin-6-yl]propyl}-cyclopentanecarboxamide

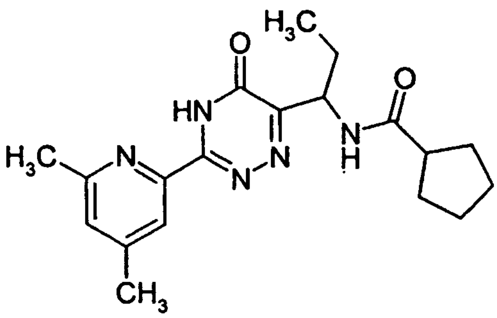

In analogy to the procedure for Example 21A, 1,28 g (6,9 mmol) 4,6-dimethyl-2-pyridinecarboximidamide hydrochloride and proportionate amounts of the other reagents are used.
Yield: 2.25 g (crude)
LC/MS (A): MS (ESI): 356 (M+H$^+$), retention time 3.48 min.

**Example 23A**

N-{1-[5-Oxo-3-(3-pyridinyl)-4,5-dihydro-1,2,4-triazin-6-yl]propyl}cyclopentanecarboxamide

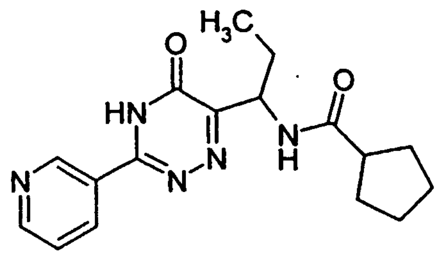

In analogy to the procedure for Example 21 A, 1,28 g (6,9 mmol) 3-pyridinecarboximidamide hydrochloride hydrochloride and proportionate amounts of the other reagents are used.
Yield: 1.4 g (32%)
$^1$H-NMR (DMSO-$d_6$, 200 MHz): δ = 0,9 (t, 3H), 1,4-1,9 (m, 10H), 2,7 (m, 1H), 4,9 (m, 1H), 7,6 (m, 1H), 8,0 (d, 1H), 8,4 (m, 1H), 8,8 (m, 1H), 9,2 (m, 1H), 14,2 (br. s, 1H) ppm.

**Example 24A**

N-{1-[5-Oxo-3-(2-thienyl)-4,5-dihydro-1,2,4-triazin-6-yl]propyl}cyclopentanecarboxamide

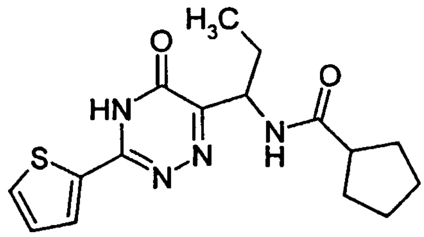

In analogy to the procedure for Example 21 A, 6,0 g (36,9 mmol) 2-thiophenecarboximidamide hydrochloride and proportionate amounts of the other reagents are used.
Yield: 0.5 g (4%)
$^1$H-NMR (DMSO-$d_6$, 200 MHz): δ = 0,9 (t, 3H), 1,4-1,9 (m, 10H), 2,7 (m, 1H), 4,9 (m, 1H), 7,3 (m, 1H), 8,0 (m, 2H), 8,1 (m, 1H), 14,2 (br. s, I H) ppm.

**Example 25A**

N-{1-[5-Oxo-3-(2-pyridinyl)-4,5-dihydro-1,2,4-triazin-6-yl]propyl}cyclopentanecarboxamide

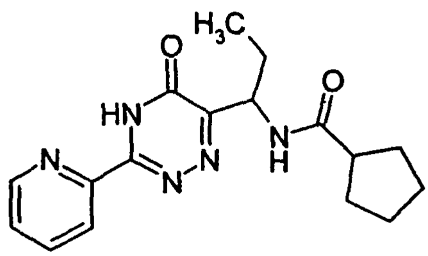

In analogy to the procedure for Example 21A, 2,8 g (17,8 mmol) 2-pyridinecarboximidamide hydrochloride and proportionate amounts of the other reagents are used.

Yield: 0.98 g (17%)
LC/MS (A): MS (ESI): 328 (M+H$^+$), retention time 3.02 min

**Example 26A**

N-{1-[5-Oxo-3-(2-furanyl)-4,5-dihydro-1,2,4-triazin-6-yl]propyl}cyclopentanecarboxamide

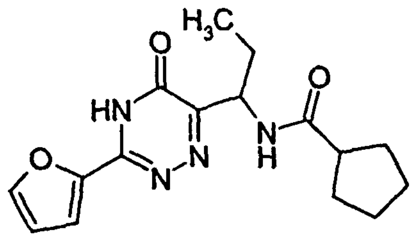

In analogy to the procedure for Example 21A, 1,3 g (8,9 mmol) 2-furancarboximidamide hydrochloride hydrochloride and proportionate amounts of the other reagents are used.
Yield: 380 mg (13%)
$^1$H-NMR (DMSO-$d_6$, 200 MHz): δ = 0,9 (t, 3H), 1,4-1,9 (m, 10H), 2,7 (m, 1H), 4,9 (m, 1H), 6,8 (m, 1H), 7,4 (d, 1H), 8,0 (m, 1H), 8,1 (m, 1H), 14,1 (br. s, 1H) ppm.

**Example 27A**

cis-4-tert-Butyl-N-{1-[5-oxo-3-(2-thienyl)-4,5-dihydro-1,2,4-triazin-6-yl]propyl}-cyclohexanecarboxamide

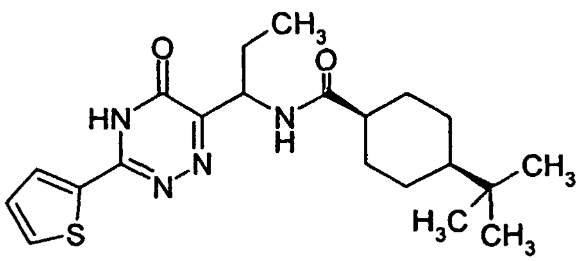

In analogy to the procedure for Example 15A, 1,00 g (4,23 mmol) of Example 14A, 0,94 g (4,65 mmol) cis-4-tert-butylcyclohexanecarbonyl chloride and proportionate amounts of the other reagents are used.
Yield: 1.6 g (94%)
LC/MS (A): MS (ESI): 403 (M+H$^+$), retention time 4.25 min

**Example 28A**

N-{1-[5-Oxo-3-(2-thienyl)-4,5-dihydro-1,2,4-triazin-6-yl]propyl}cyclobutanecarboxamide

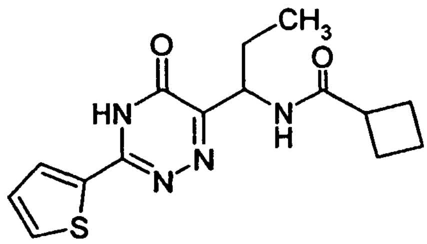

In analogy to the procedure for Example 15A, 103 mg (0,434 mmol) of Example 14A, 57 mg (0,478 mmol) cyclobutanecarbonyl chloride and proportionate amounts of the other reagents are used.
Yield: 140 mg (100%)

**Example 29A**

4-tert-Butyl-N-{1-[5-oxo-3-(2-thienyl)-4,5-dihydro-1,2,4-triazin-6-yl]propyl}cyclohexanecarboxamide

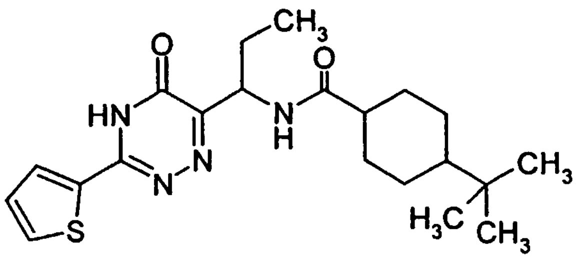

In analogy to the procedure for Example 15A, 350 mg (1,48 mmol) of Example 14A, 330 mg (1,63 mmol) 4-tert-butylcyclohexanecarbonyl chloride and proportionate amounts of the other reagents are used. A mixture of isomers is obtained.
Yield: 0.58 g (97%)
LC/MS (A): MS (ESI): 403 ($M+H^+$), retention time 4.25 min

**Example 30A**

3-Methyl-N-{1-[5-oxo-3-(2-thienyl)-4,5-dihydro-1,2,4-triazin-6-yl]propyl}butaneamide

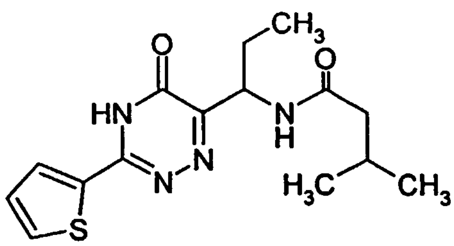

In analogy to the procedure for Example 15A, 85 mg (0,36 mmol) of Example 14A, 48 mg (0,40 mmol) 3-methylbutanoyl chloride and proportionate amounts of the other reagents are used.
Yield: 115 mg (crude)
LC/MS (A): MS (ESI): 321 ($M+H^+$), retention time 2.91 min

**Example 31A**

5-Chloro-2-thiophenecarboximidamide hydrochloride

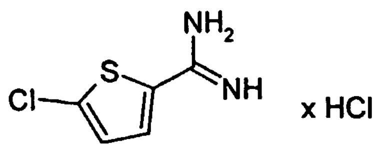

In analogy to the procedure for Example 5A, 12,5 g (66 mmol) ethyl 5-chloro-2-thiophenecarboxylate and proportionate amounts of the other reagents are used.
Yield: 9.3 g (72%)

**Example 32A**

1-Isoquinolinecarboximidamide hydrochloride

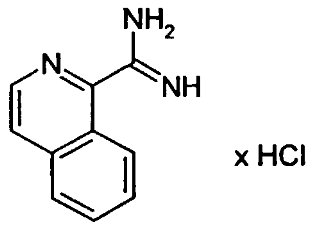

In analogy to the procedure for Example 5A, 10,0 g (64,9 mmol) 2-quinolinecarbonitrile and proportionate amounts of the other reagents are used.
Yield: 3.8 g (88%)
$^1$H-NMR (400 MHz, $CD_3OD$): δ = 7,75 (t, 1 H), 7,81 (t, 1 H), 7,97-8,03 (m, 2 H), 8,11 (d, 1 H), 8,53 (d, 1 H) ppm.

**Example 33A**

3-Bromo-2-thiophenecarboximidamide hydrochloride

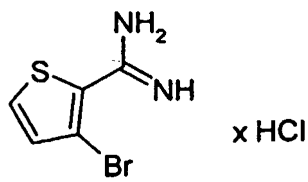

In analogy to the procedure for Example 5A, 15,0 g (79,8 mmol) 3-bromo-2-thiophenecarbonitrile and proportionate amounts of the other reagents are used.
Yield: 6.8 g (35%)

**Example 34A**

1,5-Dimethyl-1H-pyrrole-2-carboximidamide hydrochloride

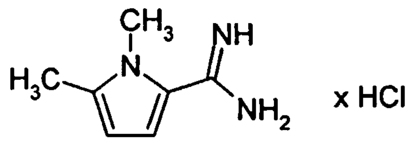

In analogy to the procedure for Example 5A, 5.0 g (41.6 mmol) 1,5-dimethyl-1H-pyrrole-2-carbonitrile and proportionate amounts of the other reagents are used.
Yield: 5.85 g (81%)
$^1$H-NMR (200 MHz, DMSO): δ = 2.3 (s, 3H), 3.6 (s, 3H), 6.1 (m, 1H), 6.7 (m, 1H), 8.7 (br.m, 3H) ppm.

**Example 35A**

3-Chloro-2-pyridinecarboximidamide hydrochloride

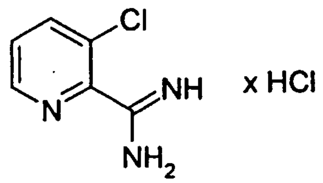

In analogy to the procedure for Example 5A, 7.8 g (56.3 mmol) 3-chloro-2-pyridinecarbonitrile and proportionate amounts of the other reagents are used.
Yield: 9.7 g (90%)
$^1$H-NMR (300 MHz, DMSO): δ = 7.7 (d/d, 1H), 8.2 (d/d, 1H), 8.6 (br.m, 4H, 8.7 (d/d, 1H) ppm.

**Example 36A**

1H-Pyrrole-2-carboximidamide hydrochloride

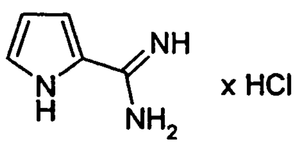

In analogy to the procedure for Example 5A, 4.9 g (53.2 mmol) 1H-pyrrole-2-carbonitrile and proportionate amounts of the other reagents are used.
Yield: 2.2 g (27%)
LC/MS (A): MS (ES+): 110 ($M^+$+H), retention time 0.45 min

**Example 37A**

3-Furancarboximidamide hydrochloride

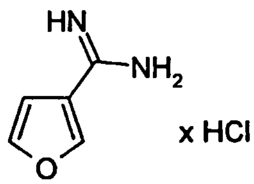

In analogy to the procedure for Example 5A, 10.0 g (71.4 mmol) ethyl 3-furoate and proportionate amounts of the other reagents are used.
Yield: 8.76 g (84%)
LC/MS (A): MS (ES+): 111 ($M^+$+H), retention time 0.40 min

**Example 38A**

1 -Methyl- 1H-pyrrole-2-carboximidamide hydrochloride

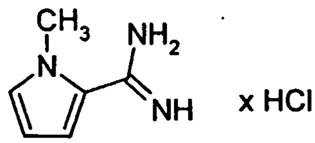

In analogy to the procedure for Example 5A, 10.0 g (79.9 mmol) 1-methyl-1H-pyrrole-2-carboxylic acid and proportionate amounts of the other reagents are used.
Yield: 6.58 g (52%)
LC/MS (A): MS (ES+): 124 ($M^+$+H), retention time 0.44 min

**Example 39A**

N-{1-[3-(5-Chloro-2-thienyl)-5-oxo-4,5-dihydro-1,2,4-triazin-6-yl]propyl}acetamide

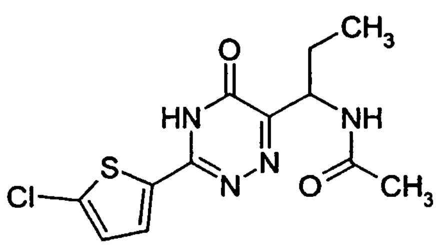

In analogy to the procedure for Example 7A, 9,26 g (47,0 mmol) 5-chloro-2-thiophenecarboximidamide hydrochloride and proportionate amounts of the other reagents are used.
Yield: 6.8 g (34%)

$^1$H-NMR (DMSO-$d_6$, 300 MHz): δ = 0,91 (t, 3 H), 1,52-1,90 (m, 5 H, s bei 1,85), 4,87 (m, 1 H), 7,34 (d, 1 H), 7,94 (d, 1 H), 8,09 (d, 1 H, NH) ppm.

**Example 40A**

N-{1-[3-(1-Isoquinolinyl)-5-oxo-4,5-dihydro-1,2,4-triazin-6-yl]propyl}acetamide

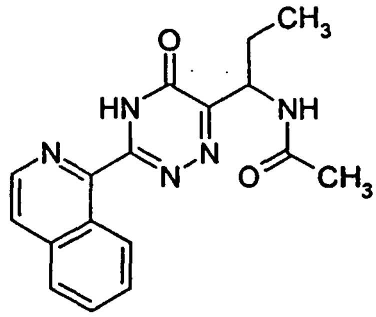

In analogy to the procedure for Example 7A, 3,7 g (17,8 mmol) 1-isoquinolinecarboximidamide hydrochloride and proportionate amounts of the other reagents are used.
Yield: 1.88 g (33%)
LC/MS (method A): retention time 1.89 min., *m*/*z* 324 $[M+H]^+$

**Example 41A**

N-{1-[3-(3-Bromo-2-thienyl)-5-oxo-4,5-dihydro-1,2,4-triazin-6-yl]propyl}acetamide

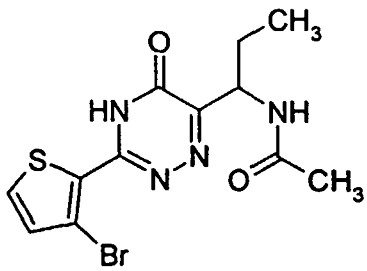

In analogy to the procedure for Example 7A, 7,5 g (31,1 mmol) 3-bromo-2-thiophenecarboximidamide hydrochloride and proportionate amounts of the other reagents are used.
Yield: 2.34 g (21%)
$^1$H-NMR ($CD_3OD$, 500 MHz): δ = 0,93 (t, 3 H), 1,58-1,96 (m, 5 H, s bei 1,92), 4,97 (m, 1 H), 7,16 (d, 1 H), 7,79 (d, 1 H) ppm.

**Example 42A**

N-{1-[5-Oxo-3-(2-pyrazinyl)-4,5-dihydro-1,2,4-triazin-6-yl]propyl}acetamide

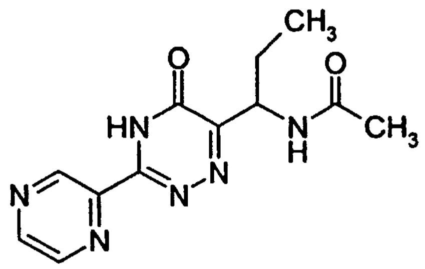

In analogy to the procedure for Example 7A, 3,0 g (18,9 mmol) 1,4-pyrazine-2-carboximidamide hydrochloride and proportionate amounts of the other reagents are used.
Yield: 1.88 g (36%)
$^1$H-NMR (DMSO-$d_6$, 200 MHz): $\delta$ = 0.9 (t, 3H), 1.6 (m, 1H), 1.8 (m, 1H), 1.9 (s, 3H), 4,9 (m, 1H), 8.2 (d, 1H), 8.7 (m, 1H), 8.9 (m, 1H), 9.4 (m, 1H) ppm.

**Example 43A**

N-{1-[3-(2-Methyl-1,3-thiazol-4-yl)-5-oxo-4,5-dihydro-1,2,4-triazin-6-yl]propyl}-acetamide

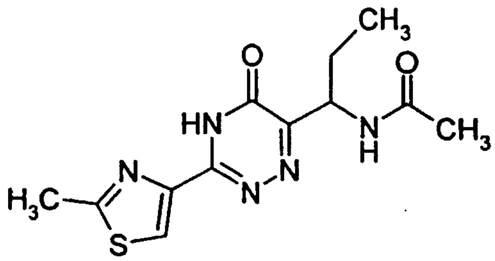

In analogy to the procedure for Example 7A, 4.5 g (25.3 mmol) 2-methyl-1,3-thiazole-4-carboximidamide hydrochloride and proportionate amounts of the other reagents are used.
Yield: 3.38 g (46%)
LC/MS (A): MS (ES+): 294 (M+H$^+$), retention time 1.51 min

**Example 44A**

N-{1-[5-Oxo-3-(1,3-thiazol-2-yl)-4,5-dihydro-1,2,4-triazin-6-yl]propyl}acetamide

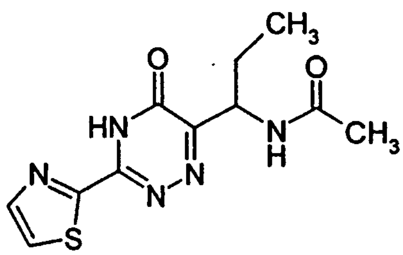

In analogy to the procedure for Example 7A, 4.95 g (30.25 mmol) 1,3-thiazole-2-carboximidamide hydrochloride

and proportionate amounts of the other reagents are used.
Yield: 3.61 g (43%)
$^1$H-NMR (DMSO-$d_6$, 400 MHz): δ = 0.9 (t, 3H), 1.6 (m, 1H), 1.8 (m, 1H), 1.9 (s, 3H), 4,9 (m, 1H), 8.2 (m, 2H), 14.6 (br.s, 1H) ppm.

**Example 45A**

N-{1-[3-(3,5-Difluoro-2-pyridinyl)-5-oxo-4,5-dihydro-1,2,4-triazin-6-yl]propyl}-acetamide

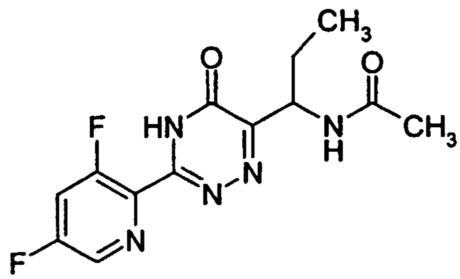

In analogy to the procedure for Example 7A, 5.00 g (25.8 mmol) 3,5-difluoro-2-pyridinecarboximidamide hydrochloride and proportionate amounts of the other reagents are used.
Yield: 2.19 g (27%)
$^1$H-NMR (DMSO-$d_6$, 300 MHz): δ = 0.9 (t, 3H), 1.6 (m, 1H), 1.8 (m, 1H), 1.9 (s, 3H), 4,9 (m, 1H), 8.1 (m, 1H), 8.2 (m, 1H), 8.7 (m, 1H), 14.1 (br.s, 1H) ppm.

**Example 46A**

N-{1-[3-(1,5-Dimethyl-1H-pyrrol-2-yl)-5-oxo-4,5-dihydro-1,2,4-triazin-6-yl]propyl}-acetamide

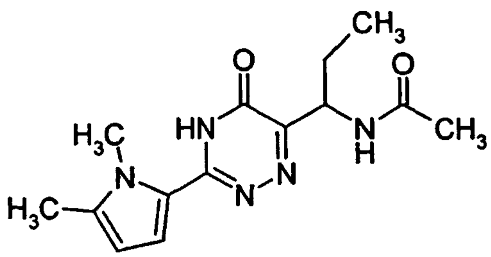

In analogy to the procedure for Example 7A, 5.80 g (33.4 mmol) of Example 34A and proportionate amounts of the other reagents are used.
Yield: 1.61 g (42%)
LC/MS (B): MS (ES+): 290 (M+H$^+$), retention time 2.54 min

**Example 47A**

N-{1-[3-(3-Bromo-2-pyridinyl)-5-oxo-4,5-dihydro-1,2,4-triazin-6-yl]propyl}-acetamide

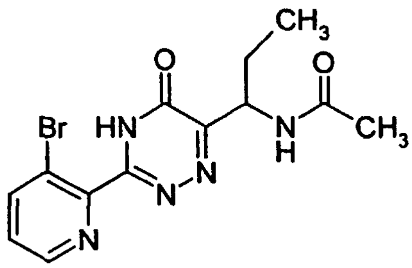

In analogy to the procedure for Example 7A, 2.59 g (10.95 mmol) 3-bromo-2-pyridinecarboximidamide hydrochloride and proportionate amounts of the other reagents are used.
Yield: 2.19 g (27%)
$^1$H-NMR (DMSO-$d_6$, 200 MHz): δ = 0.9 (t, 3H), 1.6 (m, 1H), 1.8 (m, 1H), 1.9 (s, 3H), 4,9 (m, 1H), 7.6 (m, 1H), 8.2 (br. d, 1H), 8.4 (m, 1H), 8.7 (m. 1H), 14.3 (br.s, 1H) ppm.

**Example 48A**

N-{1-[3-(3-Chloro-2-pyridinyl)-5-oxo-4,5-dihydro-1,2,4-triazin-6-yl]propyl}-acetamide

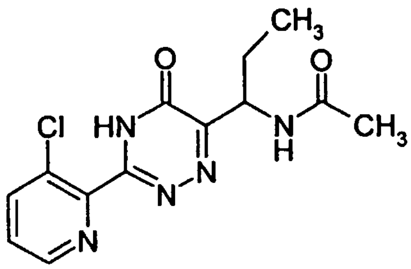

In analogy to the procedure for Example 7A, 6.00 g (31.24 mmol) of Example 35A and proportionate amounts of the other reagents are used.
Yield: 3.40 g (35%)
$^1$H-NMR (DMSO-$d_6$, 200 MHz): δ = 0.9 (t, 3H), 1.6 (m, 1H), 1.8 (m, 1H), 1.9 (s, 3H), 4,9 (br. m, 1H), 7.7 (d/d, 1H), 8.2 (d/d, 1H), 8.7 (d/d, 1H), 14.3 (br.s, 1H) ppm.

**Example 49A**

N-{1-[5-Oxo-3-(1H-pyrrol-2-yl)-4,5-dihydro-1,2,4-triazin-6-yl]propyl}acetamide

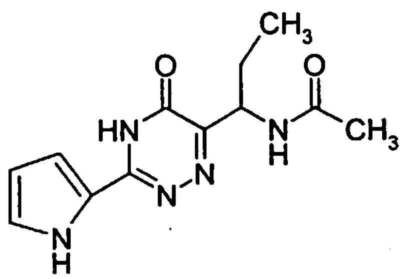

In analogy to the procedure for Example 7A, 6.15 g (42.24 mmol) of Example 36A and proportionate amounts of the other reagents are used.
Yield: 3.98 g (36%)
LC/MS (A): MS (ES+): 262 ($M+H^+$), retention time 1.61 min

**Example 50A**

N-{1-[3-(3-Furyl)-5-oxo-4,5-dihydro-1,2,4-triazin-6-yl]propyl}acetamide

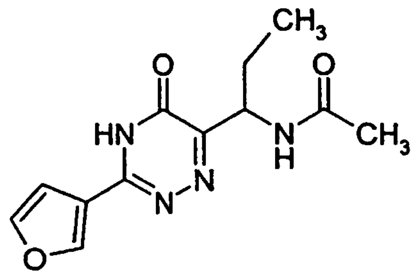

In analogy to the procedure for Example 7A, 8.76 g (59.8 mmol) of Example 37A and proportionate amounts of the other reagents are used.
Yield: 4.26 g (27%)
LC/MS (A): MS (ES+): 263 ($M+H^+$), retention time 1.55 min

**Example 51A**

N- {1-[3-(1-Methyl-1H-pyrrol-2-yl)-5-oxo-4,5-dihydro-1,2,4-triazin-6-yl]propyl}-acetamide

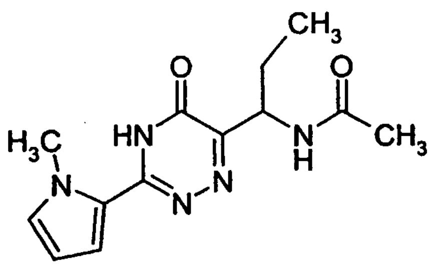

In analogy to the procedure for Example 7A, 6.58 g (41.22 mmol) of Example 38A and proportionate amounts of the other reagents are used.
Yield: 2.88 g (25%)
LC/MS (A): MS (ES+): 276 ($M+H^+$), retention time 1.73 min

**Example 52A**

N-{1-[5-Oxo-3-(3-pyridinyl)-4,5-dihydro-1,2,4-triazin-6-yl]propyl}acetamide

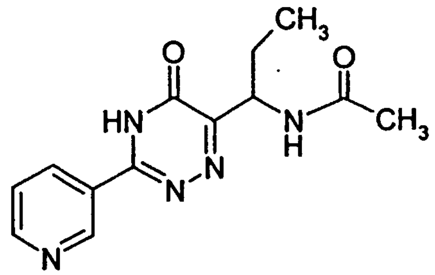

In analogy to the procedure for Example 7A, 15,0 g (0,1 mol) 3-pyridincarboximidamide hydrochloride and proportionate amounts of the other reagents are used.
Yield: 13.1 g (50%)
$^1$H-NMR ($d_6$-DMSO, 200 MHz): δ = 0.9 (t, 3H), 1.6 (m, 2H), 1.8 (m, 4H); 4.9 (m, 1H); 7.6 (m, 1H); 8.2 (m, 1H); 8.4 (m, 1H), 8.8 (m, 1H), 9.2 (m, 1H), 14.5 (bs, 1H) ppm.

**Example 53A**

N-{1-[5-Oxo-3-(2-pyridinyl)-4,5-dihydro-1,2,4-triazin-6-yl]propyl}acetamide

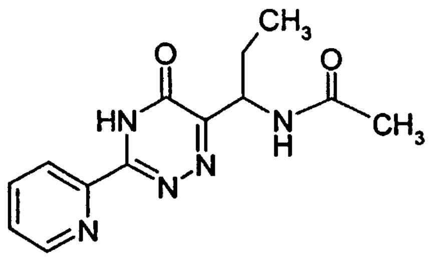

In analogy to the procedure for Example 7A, 6,0 g (38,1 mmol) 2-pyridincarboximidamide hydrochloride and proportionate amounts of the other reagents are used.
Yield: 5.6 g (54%)
$^1$H-NMR ($d_6$-DMSO, 200 MHz): δ = 0.9 (t, 3H), 1.7 (m, 2H), 1.9 (s, 3H); 4.9 (m, 1H); 7.5 (bs); 7.7 (m, 1H); 8.2 (m, 2H), 8.8 (m, 1H) ppm.

**Example 54A**

N-{1-[5-Oxo-3-(4-pyridinyl)-4,5-dihydro-1,2,4-triazin-6-yl]propyl}acetamide

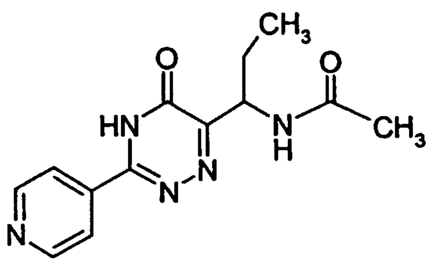

In analogy to the procedure for Example 7A, 10,0 g (63,5 mmol) 4-pyridincarboximidamide hydrochloride and proportionate amounts of the other reagents are used.
Yield: 10.5 g (61 %)
$^1$H-NMR ($d_6$-DMSO, 200 MHz): δ = 1,0 (t, 3H), 1.8 (m, 2H), 2.0 (s, 3H); 5.0 (m, 1H); 7.8 (m, 2H); 8.1 (m, 2H), 8.8 (m, 2H) ppm.

**Example 55A**

N-{1-[3-(2,5-Dichloro-1,3-thiazol-4-yl)-5-oxo-4,5-dihydro-1,2,4-triazin-6-yl]propyl}-acetamide

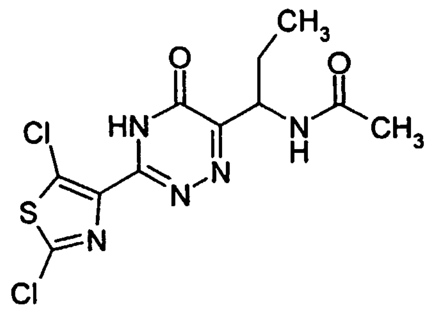

In analogy to the procedure for Example 7A, 5,0 g (21,5 mmol) 2,5-dichloro-1,3-thiazole-4-carboximidamide hydrochloride and proportionate amounts of the other reagents are used.
Yield: 600 mg (8%)
$^1$H-NMR ($d_6$-DMSO, 300 MHz): δ = 0,9 (t, 3H), 1.6 (m, 2H), 1.9 (s, 3H); 4.9 (m, 1H); 8.1 (m, 1H), 14.2 (bs, 1H) ppm.

**Example 56A**

N-{1-[3-(2-Furyl)-5-oxo-4,5-dihydro-1,2,4-triazin-6-yl]propyl}acetamide

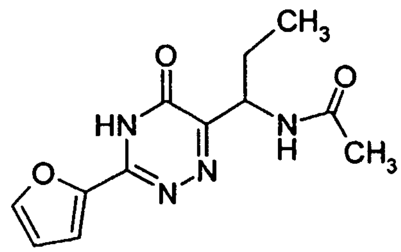

In analogy to the procedure for Example 7A, 5,0 g (21,5 mmol) 2-furancarboximidamide hydrochloride and proportionate amounts of the other reagents are used.
Yield: 2.8 g (31 %)
$^1$H-NMR ($d_6$-DMSO, 200 MHz): δ = 0,9 (t, 3H), 1.6 (m, 1H), 1.8 (m, 1H), 1.9 (s, 3H); 4.9 (m, 1H); 6.8 (m, 1H); 7.5 (m, 1H), 8.1 (m, 2H); 14.1 (bs, 1H) ppm.

**Example 57A**

6-(1-Aminopropyl)-3-(5-chloro-2-thienyl)-1,2,4-triazin-5(4H)-one

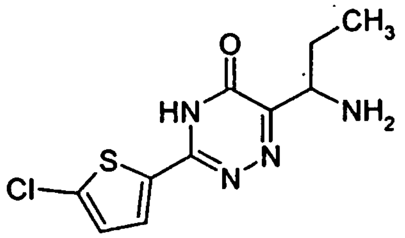

In analogy to the procedure for Example 11A, 1,7 g (2,14 mmol) N-{1-[3-(5-chloro-2-thienyl)-5-oxo-4,5-dihydro-1,2,4-triazin-6-yl]propyl}acetamide and proportionate amounts of the other reagents are used.
Yield: 0.35 g (61%)
$^1$H-NMR ($CD_3OD$, 400 MHz): δ = 1,01 (t, 3 H), 1,90-2,19 (m, 2 H), 4,45 (t, 1 H), 7,01 (d, 1 H), 7,68 (d, 1 H) ppm.

**Example 58A**

6-(1-Aminopropyl)-3-(1-isoquinolinyl)-1,2,4-triazin-5(4H)-one

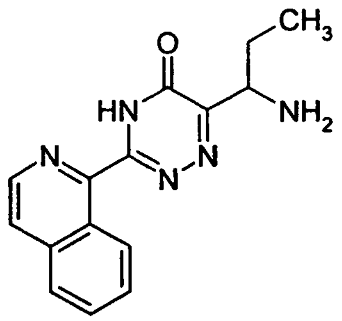

In analogy to the procedure for Example 11A, 1,88 g (3,66 mmol) N-{1-[3-(1-isoquinolinyl)-5-oxo-4,5-dihydro-1,2,4-triazin-6-yl]propyl}acetamide and proportionate amounts of the other reagents are used.
Yield: 0.5 g (48%)
$^1$H-NMR ($CD_3OD$, 400 MHz): δ = 1,08 (t, 3 H), 1,99-2,27 (m, 2 H), 4,59 (t, 1 H), 7,66 (t, 1 H), 7,81 (t, 1 H), 7,94 (d, 1 H), 8,02 (d, 1 H), 8,20 (d, 1 H), 8,53 (d, 1 H) ppm.

**Example 59A**

6-(1-Aminopropyl)-3-(3-bromo-2-thienyl)-1,2,4-triazin-5(4H)-one

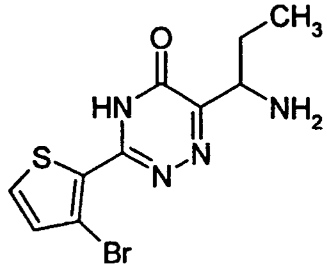

In analogy to the procedure for Example 11 A, 2,33 g (6,52 mmol) N-{1-[3-(3-bromo-2-thienyl)-5-oxo-4,5-dihydro-1,2,4-triazin-6-yl]propyl}acetamide and proportionate amounts of the other reagents are used.
Yield: 1.04 g (51%)
$^1$H-NMR ($CD_3OD$, 400 MHz): δ = 1,02 (t, 3 H), 1,92-2,21 (m, 2 H), 4,48 (t, 1 H), 7,10 (d, 1 H), 7,56 (d, 1 H) ppm.

**Example 60A**

6-(1-Aminopropyl)-3-(2-pyrazinyl)-1,2,4-triazin-5(4H)-one

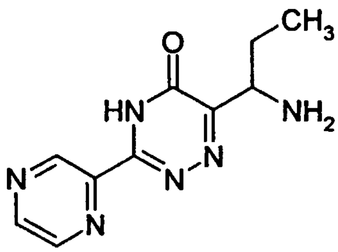

In analogy to the procedure for Example 11A, 1.88 g (6,9 mmol) of Example 42A and proportionate amounts of the other reagents are used.
Yield: 1.5 g (93%)
LC/MS (A): MS (ES+): 233 ($M+H^+$), retention time 0.37 min

**Example 61A**

6-(1-Aminopropyl)-3-(2-methyl-1,3-thiazol-4-yl)-1,2,4-triazin-5(4H)-one

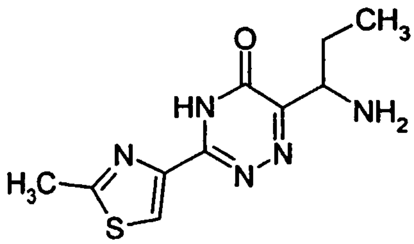

In analogy to the procedure for Example 11A, 3.35 g (11.42 mmol) of Example 43A and proportionate amounts of the other reagents are used.
Yield: 1.51 g (53%)
LC/MS (A): MS (ES+): 252 ($M+H^+$), retention time 0.48 min

**Example 62A**

6-(1-Aminopropyl)-3-(1,3-thiazol-2-yl)-1,2,4-triazin-5(4H)-one

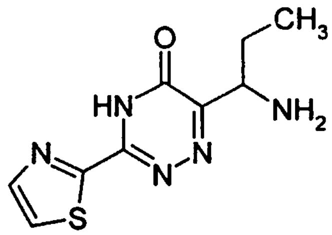

In analogy to the procedure for Example 11A, 3.60 g (12.9 mmol) of Example 44A and proportionate amounts of the other reagents are used.
Yield: 1.76 g (57%)
$^1$H-NMR (DMSO-$d_6$, 200 MHz): $\delta$ = 0.9 (t, 3H), 1.9 (m, 2H), 4,3 (t, 1H), 7.8 (d, 1H), 7.9 (d, 1H), 8.2 (br. m, 3H).

**Example 63A**

6-(1-Aminopropyl)-3-(3,5-difluoro-2-pyridinyl)-1,2,4-triazin-5(4H)-one

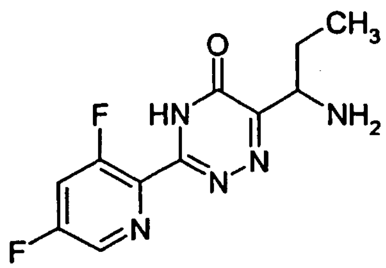

In analogy to the procedure for Example 11A, 2.15 g (6.9 mmol) of Example 45A and proportionate amounts of the other reagents are used.
Yield: 0.68 g (37%)
LC/MS (A): MS (ES+): 268 (M+H$^+$), retention time 0.44 min

**Example 64A**

6-(1-Aminopropyl)-3-(1,5-dimethyl-1H-pyrrol-2-yl)-1,2,4-triazin-5(4H)-one

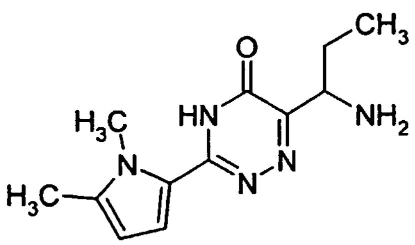

In analogy to the procedure for Example 11A, 3.67 g (12.7 mmol) of Example 46A and proportionate amounts of the other reagents are used.

Yield: 1.69 g (54%)
LC/MS (A): MS (ES+): 248 ($M+H^+$), retention time 1.31 min

**Example 65A**

6-(1-Aminopropyl)-3-(3-bromo-2-pyridinyl)-1,2,4-triazin-5(4H)-one

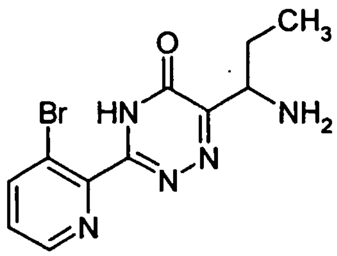

In analogy to the procedure for Example 11A, 1.60 g (4.54 mmol) of Example 47A and proportionate amounts of the other reagents are used.
Yield: 0.48 g (34%)
$^1$H-NMR (DMSO-$d_6$, 300 MHz): $\delta$ = 0.9 (t, 3H), 1.9 (m, 1H), 2.0 (m, 1H), 4,3 (t, 1H), 7.4 (m, 1H), 8.0 (br. s, 3H), 8.1 (m, 1H), 8.6 (m, 1H) ppm.

**Example 66A**

6-(1-Aminopropyl)-3-(3-chloro-2-pyridinyl)-1,2,4-triazin-5(4H)-one

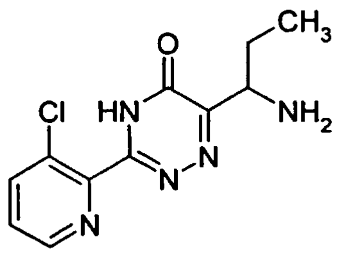

In analogy to the procedure for Example 11A, 3.40 g (11.05 mmol) of Example 48A and proportionate amounts of the other reagents are used.
Yield: 1.24 g (42%)
$^1$H-NMR (DMSO-$d_6$, 300 MHz): $\delta$ = 0.9 (t, 3H), 1.9 (m, 2H), 4,3 (t, 1H), 7.5 (d/d, 1H), 8.0 (d/d, 1H), 8.0 (br.s, 3H), 8.5 (d/d, 1H) ppm.

**Example 67A**

6-(1-Aminopropyl)-3-(1 H-pyrrol-2-yl)-1,2,4-triazin-5(4H)-one

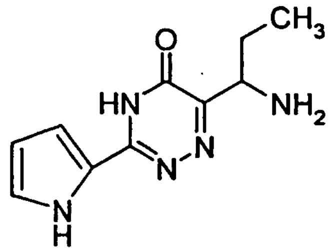

In analogy to the procedure for Example 11A, 3.98 g (15.23 mmol) of Example 49A and proportionate amounts of the other reagents are used.
Yield: 1.82 g (54%)
$^{1}$H-NMR (DMSO-$d_6$, 300 MHz): $\delta$ = 0.9 (t, 3H), 1.9 (m, 1H), 2.0 (m, 1H), 4,2 (t, 1H), 6.2 (m, 1H, 6.9 (m, 2H), 8.4 (br. s, 3H), 11.6 (br. s, 1H) ppm.

**Example 68A**

6-(1-Aminopropyl)-3-(3-furyl)-1,2,4-triazin-5(4H)-one

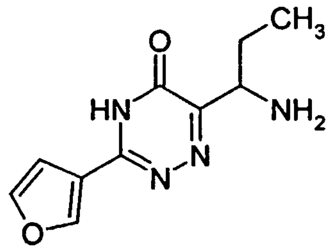

In analogy to the procedure for Example 11A, 4.26 g (16.24 mmol) of Example 50A and proportionate amounts of the other reagents are used. The product is used for the next step without further purification.
LC/MS (B): MS (ES+): 221 (M+H$^{+}$), retention time 0.35 min

**Example 69A**

6-(1-Aminopropyl)-3-(1-methyl-1H-pyrrol-2-yl)-1,2,4-triazin-5(4H)-one

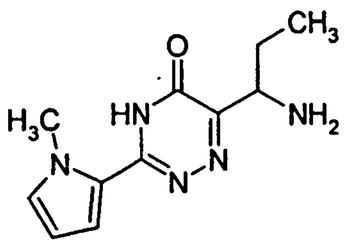

In analogy to the procedure for Example 11A, 2.88 g (10.46 mmol) of Example 51A and proportionate amounts

of the other reagents are used. The product is used for the next step without further purification.
LC/MS (B): MS (ES+): 234 ($M+H^+$), retention time 0.40 min

**Example 70A**

6-(1-Aminopropyl)-3-(3-pyridinyl)-1,2,4-triazin-5(4H)-one

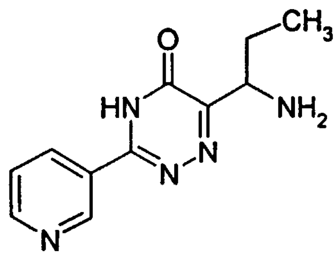

In analogy to the procedure for Example 11A, 3,40 g (10 mmol) of Example 52A and proportionate amounts of the other reagents are used. The compound is used without further purification.
LC/MS (A): MS (ESI): 232 ($M+H^+$), retention time 0.37 min
$^1$H-NMR ($d_6$-DMSO, 200 MHz): $\delta$ = 0,9 (t, 3H), 1.9 (m, 2H), 4.3 (m, 1H), 7.5 (br. s), 8.1-9.4 (m) ppm.

**Example 71A**

6-(1-Aminopropyl)-3-(2-pyridinyl)-1,2,4-triazin-5(4H)-one

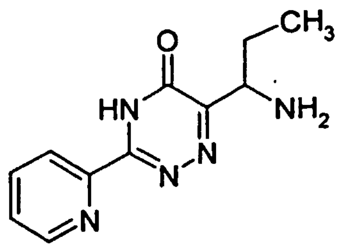

In analogy to the procedure for Example 11A, 7,60 g (27,8 mmol) of Example 53A and proportionate amounts of the other reagents are used. The compound is used without further purification.
LC/MS (A): MS (ESI): 232 ($M+H^+$), retention time 0.35 min
$^1$H-NMR ($d_6$-DMSO, 200 MHz): $\delta$ = 0,9 (t, 3H), 1.9 (m, 2H), 4.3 (m, 1H), 7.8 (br. s), 8.0 (m, 1H), 8.3 (m, 1H), 8.8 (m, 1H) ppm.

**Example 72A**

6-(1-Aminopropyl)-3-(4-pyridinyl)-1,2,4-triazin-5(4H)-one

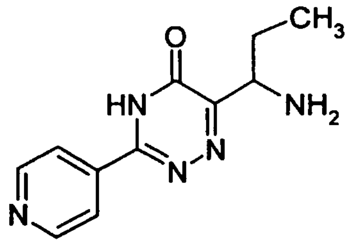

In analogy to the procedure for Example 11A, 4,50 g (16,5 mmol) of Example 54A and proportionate amounts of the other reagents are used.
Yield: 3.1 g (81 %)
LC/MS (A): MS (ESI): 232 ($M+H^+$), retention time 0.34 min
$^1$H-NMR ($d_6$-DMSO, 200 MHz): $\delta$ = 0,9 (t, 3H), 1.9 (m, 2H), 4.3 (m, 1H), 7.5 (br. s), 8.1 (m, 2H), 8.7 (m, 2H) ppm.

**Example 73A**

6-(1-Aminopropyl)-3-(2,5-dichloro-1,3-thiazol-4-yl)-1,2,4-triazin-5(4H)-one

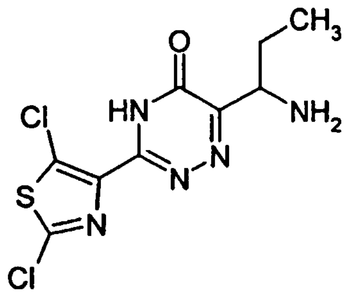

In analogy to the procedure for Example 11A, 200 mg (0,57 mmol) of Example 55A and proportionate amounts of the other reagents are used.
Yield: 150 mg (85%)
LC/MS (B): MS (ESI): 306 ($M+H^+$), retention time 0.35 min

**Example 74A**

6-(1-Aminopropyl)-3-(2-furyl)-1,2,4-triazin-5(4H)-one

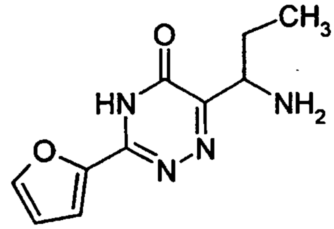

In analogy to the procedure for Example 11A, 2,60 g (9,91 mmol) of Example 56A and proportionate amounts of the other reagents are used. The compound is used without further purification.

LC/MS (A): MS (ESI): 221 (M+H+), retention time 0.33 min
$^1$H-NMR ($d_6$-DMSO, 200 MHz): δ = 0.8 (t, 3H), 1.7 (m, 2H), 3.7 (m, 1H), 6.5 (m, 1H), 6.9 (m, 1H), 7.7 (m, 1H) ppm.

**Example 75A**

N-{1-[5-Oxo-3-(2-thienyl)-4,5-dihydro-1,2,4-triazin-6-yl]propyl}-3-(trifluoromethyl)-cyclohexanecarboxamide

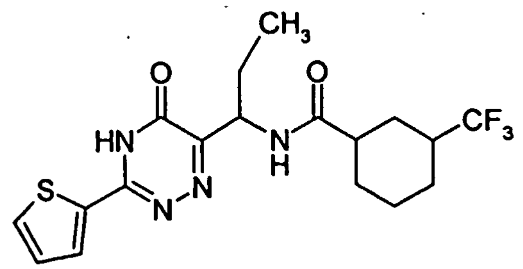

83 mg (0,42 mmol, 1 equiv.) 3-trifluoromethylcyclohexanecarboxylic acid are suspended in dichloromethane at 0°C and 62 mg (0,456 mmol, 1,05 equiv.) 1-hydroxy-1H-benzotriazol and 87 mg (0,456 mmol, 1,05 equiv.) 1-(3-dimethylaminopropyl)-3-ethylcarbodiimide hydrochloride are consecutively added. After stirring at room temperature for 30 min, 100 mg (0,42 mmol) of Example 14A are added. The reaction mixture is stirred at room temperature for 2 hours. The mixture is diluted with dichloromethane, washed twice with 1N sulfuric acid and once with saturated sodium bicarbonate solution, dried over magnesium sulfate and evaporated to dryness *in vacuo*. The product is used without further purification.
Yield: 160 mg (91%)
LC/MS (B): MS (ESI): 415 (M+H+), retention time 3.63 min

**Example 76A**

4-Methyl-N-{1-[5-oxo-3-(2-thienyl)-4,5-dihydro-1,2,4-triazin-6-yl]propyl}cyclohexanecarboxamide

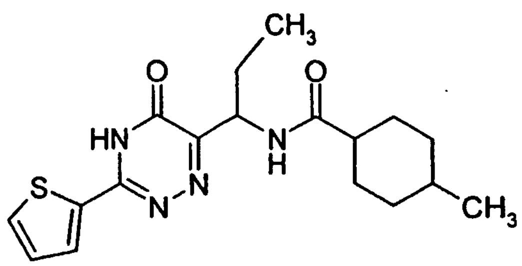

In analogy to the procedure for Example 75A, 103 mg (0,43 mmol) of Example 14A, 62 mg (0,43 mmol) 4-methylcyclohexanecarboxylic acid and proportionate amounts of the other reagents are used.
Yield: 150 mg (95%)
LC/MS (B): MS (ESI): 361 (M+H+), retention time 3.59 min

**Example 77A**

2-Cyclohexyl-N-{1-[5-oxo-3-(2-thienyl)-4,5-dihydro-1,2,4-triazin-6-yl]propyl}-acetamide

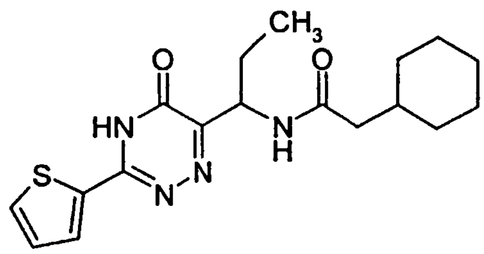

In analogy to the procedure for Example 15A, 100 mg (0,42 mmol) of Example 14A, 70 mg (0,47 mmol) cyclohexylacetyl chloride and proportionate amounts of the other reagents are used.
Yield: 150 mg (98%)
LC/MS (B): MS (ESI): 361 ($M+H^+$), retention time 3.51 min

**Example 78A**

1,4-Dimethyl-N-{1-[5-oxo-3-(2-thienyl)-4,5-dihydro-1,2,4-triazin-6-yl]propyl}cyclohexanecarboxamide

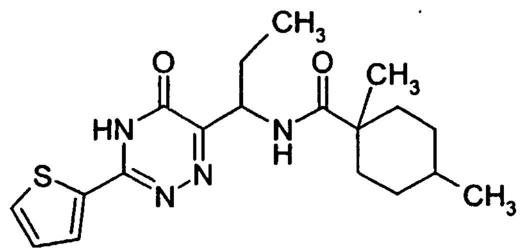

In analogy to the procedure for Example 15A, 100 mg (0,42 mmol) of Example 14A, 80 mg (0,47 mmol) 1,4-dimethylcyclohexanecarbonyl chloride and proportionate amounts of the other reagents are used.
Yield: 150 mg (94%)
LC/MS (B): MS (ESI): 375 ($M+H^+$), retention time 3.88 min

**Example 79A**

N-[1-(3-(2-Thienyl)-5-oxo-4,5-dihydro-1,2,4-triazin-6-yl)propyl]-1-adamantanecarboxamide

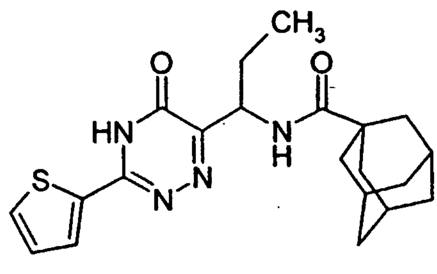

In analogy to the procedure for Example 15A, 100 mg (0,43 mmol) of Example 14A, 95 mg (0,48 mmol) 1-adamantanecarbonyl chloride and proportionate amounts of the other reagents are used.
Yield: 160 mg (92%)

LC/MS (B): MS (ESI): 399 (M+H+), retention time 3.90 min

**Example 80A**

4-tert-Butyl-N-{1-[5-oxo-3-(3-pyridinyl)-4,5-dihydro-1,2,4-triazin-6-yl]propyl}-cyclohexanecarboxamide

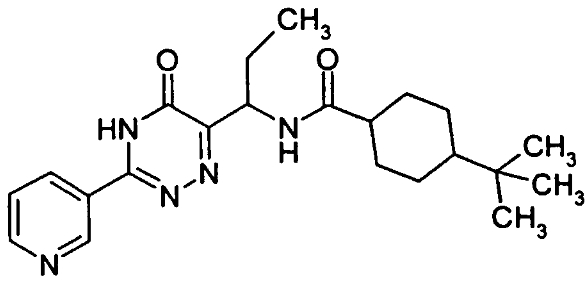

In analogy to the procedure for Example 15A, 250 mg (1,08 mmol) of Example 70A, 240 mg (1,19 mmol) 4-tert-butylcyclohexanecarbonyl chloride and proportionate amounts of the other reagents are used.
Yield: 200 mg (47%)
LC/MS (B): MS (ESI): 398 (M+H+), retention time 3.79 min

**Example 81A**

4-cis-tert-Butyl-N-{1-[5-oxo-3-(2-pyridinyl)-4,5-dihydro-1,2,4-triazin-6-yl]propyl}-cyclohexanecarboxamide

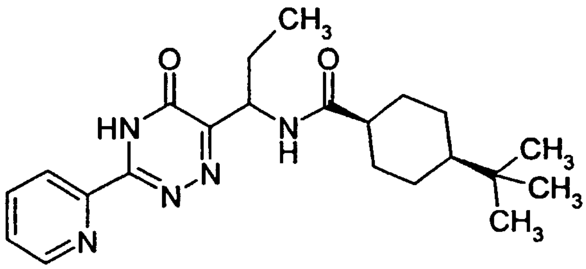

In analogy to the procedure for Example 15A, 200 mg (0,86 mmol) of Example 71A, 190 mg (0,95 mmol) 4-cis-tert-butylcyclohexanecarbonyl chloride and proportionate amounts of the other reagents are used.
Yield: 300 mg (87%)
LC/MS (B): MS (ESI): 398 (M+H+), retention time 4.21 min

**Example 82A**

4-cis-tert-Butyl-N- {1-[5-oxo-3-(4-pyridinyl)-4,5-dihydro-1,2,4-triazin-6-yl]propyl}-cyclohexanecarboxamide

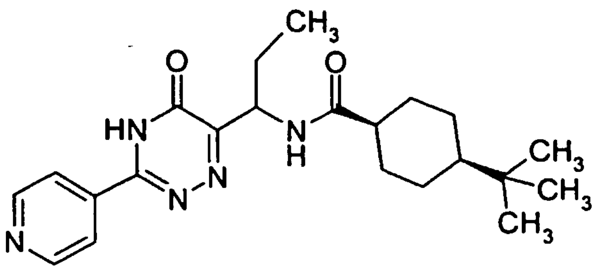

In analogy to the procedure for Example 15A, 200 mg (0,86 mmol) of Example 72A, 190 mg (0,95 mmol) 4-cis-tert-butylcyclohexanecarbonyl chloride and proportionate amounts of the other reagents are used.
Yield: 300 mg (87%)
LC/MS (B): MS (ESI): 398 ($M+H^+$), retention time 3.78 min

**Example 83A**

4-tert-Butyl-N-{1-[3-(2,5-dichloro-1,3-thiazol-4-yl)-5-oxo-4,5-dihydro-1,2,4-triazin-6-yl]propyl}cyclohexanecarboxamide

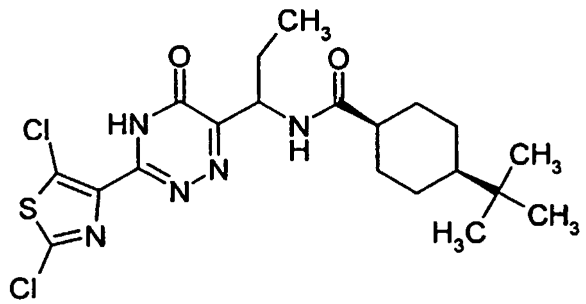

In analogy to the procedure for Example 15A, 150 mg (0,49 mmol) of Example 73A, 110 mg (0,54 mmol) 4-cis-tert-butylcyclohexanecarbonyl chloride and proportionate amounts of the other reagents are used.
Yield: 100 mg (43%)
MS (ESI): 473 ($M+H^+$)

**Example 84A**

4-tert-Butyl-N-{1-[3-(2-furyl)-5-oxo-4,5-dihydro-1,2,4-triazin-6-yl]propyl}-cyclohexanecarboxamide

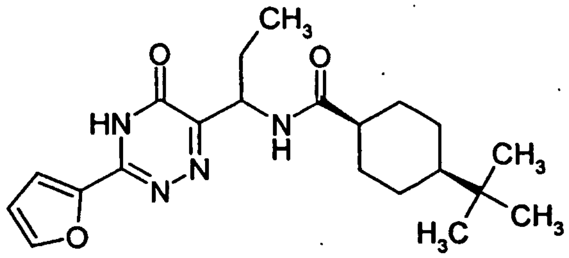

In analogy to the procedure for Example 15A, 250 mg (1,14 mmol) of Example 74A, 250 mg (1,25 mmol) 4-cis-tert-butylcyclohexanecarbonyl chloride and proportionate amounts of the other reagents are used.
Yield: 300 mg (68%)
LC/MS (B): MS (ESI): 387 ($M+H^+$), retention time 4.00 min

**Example 85A**

cis-4-*tert*-Butylcyclohexanecarboxylic acid

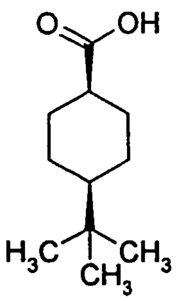

A preparative HPLC separation of cis- and trans-4-*tert*-butylcyclohexanecarboxylic acid was carried out under the following conditions:

| | |
|---|---|
| Feed: | 10 g isomeric mixture of cis- and trans-4-*tert*-butyl-cyclohexanecarboxylic acid dissolved in 500 ml iso-hexane (80%) / *tert*-butylmethylether (20%) |
| Column: | 330 x 100 mm; Self Packing Device NW 100; Merck |
| Stationary phase: | LiChrospher Si 60, 12 μm, Merck |
| Mobile phase: | iso-hexane / *tert*-butylmethylether (4/1 v/v) + 0.25 vol-% acetic acid |
| Flow: | 150 ml/min |
| Injection volume: | 70 ml (= 1.4 g compound) |
| Wave length: | 210 nm |
| Temperature: | 25°C |

The sample run on this column was repeatedly injected every 30 minutes. The cis-isomer is the first eluting compound.
cis-isomer:
mp: 118°C
$^1$H-NMR (300 MHz, DMSO): δ = 0.9 (t, 3 H), 1.0 (m, 3 H), 1.4 (m, 2 H), 1.6 (m, 1 H), 2.1 (m, 2 H), 2.5 (m, 1 H), 12.0 (s, 1 H) ppm.
trans-isomer:
mp: 172°C
$^1$H-NMR (300 MHz, DMSO): δ = 0.9 (t, 3 H), 1.0 (m, 3 H), 1.3 (m, 2 H), 1.7 (m, 1 H), 1.9 (m, 2 H), 2.1 (m, 1 H), 11.9 (s,

1 H) ppm.

**Example 86A**

cis-4-*tert*-Butylcyclohexanecarbonyl chloride

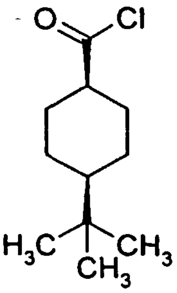

2.0 g (10.85 mmol) cis-4-*tert*-Butylcyclohexanecarboxylic acid are dissolved in 50 ml dichloromethane, 1.65 g (13.02 mmol) ethanedioyl dichloride are added and the solution is stirred at room temperature for one hour. The mixture is then stirred at reflux for two hours and, after cooling down to room temperature, evaporated to dryness *in vacuo*. The residue is then dissolved in toluene two times and again evaporated to dryness *in vacuo*. The residue is used in the next step without further purification.

__Preparation Examples__

**Example 1**

7-Cyclobutyl-5-ethyl-2-(2-phenyl-1,3-thiazol-4-yl)imidazo[5,1-f][1,2,4]triazin-4(3H)-one

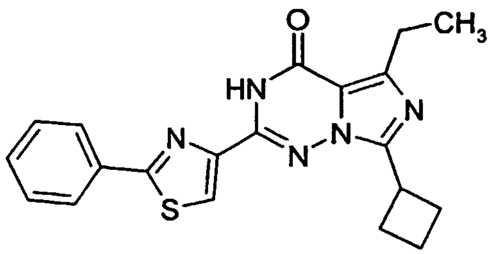

202 mg (0,51 mmol, 1 equiv.) of Example 16A are suspended in 10 ml dichloroethane, and 117 mg (0,77 mmol, 1,5 equiv.) phosphoroxychloride are added. The mixture is stirred at reflux for 3 hours. After cooling down to room temperature, ethyl acetate and saturated $NaHCO_3$ (aq) are added. The organic phase is washed with saturated $NaHCO_3$ (aq), water and brine, dried over sodium sulfate and evaporated to dryness *in vacuo*. The product is purified by chromatography (flash or column chromatography or preparative HPLC).
Yield: 108 mg (56%)
$^1$H-NMR (DMSO-$d_6$, 300 MHz): $\delta$ = 1,2 (t, 3H), 2,0 (m, 2H), 2,4 (m, 4H), 2,9 (q, 2H, 4,0 (m, 1H, 7,5 (m, 3H), 8,2 (m, 2H), 8,5 (s, 1H), 11,7 (s, 1H) ppm.

**Example 2**

7-Cyclopentyl-5-ethyl-2-(2-phenyl-1,3-thiazol-4-yl)imidazo[5,1-f][1,2,4]triazin-4(3H)-one

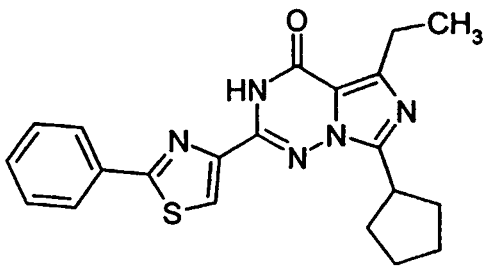

In analogy to the procedure for Example 1, 155 mg (0,38 mmol) of Example 15A, 87 mg (0,57 mmol) phosphoric trichloride are stirred at reflux for 3 hours, proportionate amounts of the solvents are used.
Yield: 80 mg (54%)
$^1$H-NMR (DMSO-$d_6$, 300 MHz): δ = 1,2 (t, 3H), 1,7 (m, 2H), 1,8 (m, 4H), 2,1 (m,2H), 2,9 (q, 2H), 3,6 (m, 1H), 7,5 (m, 3H), 8,2 (m, 2H), 8,5 (s, 1H), 11,7 (s, 1H) ppm.

**Example 3**

7-Cyclopentyl-5-ethyl-2-(3-thienyl)imidazo[5,1-f][1,2,4]triazin-4(3H)-one

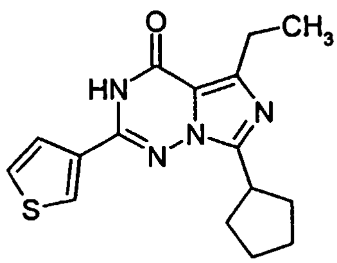

In analogy to the procedure for Example 1, 550 mg (1,65 mmol) of Example 17A, 380 mg (2,48 mmol) phosphoric trichloride are stirred at reflux for 3 hours, proportionate amounts of the solvents are used.
Yield: 80 mg (15%)
$^1$H-NMR (DMSO-$d_6$, 200 MHz): δ = 1,2 (t, 3H), 1,8 (m, 6H), 2,1 (m,2H), 2,9 (q, 2H), 3,6 (m, 1H), 7,7 (m, 2H), 8,5 (m, 1H), 11,7 (s, 1H) ppm.

**Example 4**

7-Cyclobutyl-5-ethyl-2-(3-thienyl)imidazo[5,1-f][1,2,4]triazin-4(3H)-one

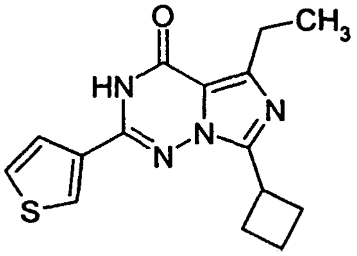

In analogy to the procedure for Example 1, 530 mg (1,66 mmol) of Example 18A, 383 mg (2,50 mmol) phosphoric trichloride are stirred at reflux for 3 hours, proportionate amounts of the solvents are used.
Yield: 47 mg (9%)
$^1$H-NMR (DMSO-$d_6$, 200 MHz): δ = 1,2 (t, 3H), 1,8 (m, 1H), 2,1 (m, 1H), 2,4 (m, 4H), 2,9 (q, 2H), 4,0 (m, 1H), 7,7 (m, 2H), 8,5 (m, 1H), 11,8 (s, 1H) ppm.

**Example 5 and Example 6**

7-(4-tert-Butylcyclohexyl)-5-ethyl-2-(3-thienyl)imidazo[5,1-f][1,2,4]triazin-4(3H)-one

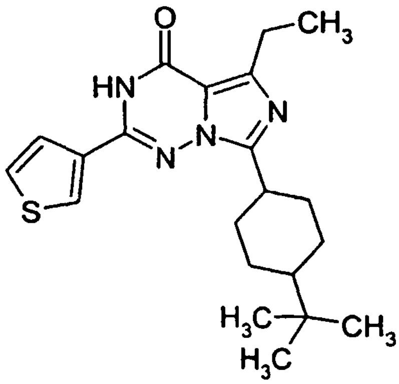

In analogy to the procedure for Example 1, 680 mg (1,69 mmol) of Example 19A, 389 mg (2,53 mmol) phosphoric trichloride are stirred at reflux for 3 hours, proportionate amounts of the solvents are used. The isomers are separated by chromatography.
Yield: 18 mg (3%) cis-isomer
90 mg (14%) trans-isomer
cis-isomer (Example 5):
$^1$H-NMR (DMSO-$d_6$, 300 MHz): δ = 0,8 (s, 9H), 1,1 (m, 1H), 1,2 (t, 3H), 1,6 (m, 3H), 1,7 (m, 3H), 2,2 (m, 2H), 2,9 (m, 2H), 3,5 (m, 1H), 7,7 (m, 1H), 7,7 (m, 1H), 8,5 (m, 1H), 11,7 (s, 1H) ppm.
trans-isomer (Example 6):
$^1$H-NMR (DMSO-$d_6$, 300 MHz): δ = 0,9 (s, 9H), 1,1 (m, 2H), 1,2 (t, 3H), 1,6 (m, 2H), 1,8 (m, 2H), 2,0 (m, 2H), 2,9 (m, 2H), 3,1 (m, 1H), 7,7 (m, 1H), 7,7 (m, 2H), 11,8(s, 1H) ppm.

**Example 7**

7-Cyclopentyl-5-ethyl-2-(2-quinolinyl)imidazo[5,1-f][1,2,4]triazin-4(3H)-one

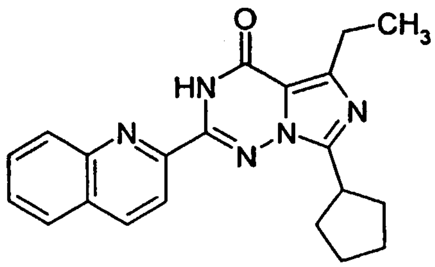

In analogy to the procedure for Example 1, 280 mg (0,73 mmol) N-{1-[5-oxo-3-(2-quinolinyl)-4,5-dihydro-1,2,4-triazin-6-yl]propyl}cyclopentanecarboxamide, 560 mg (3,64 mmol) phosphoric trichloride are stirred at reflux for 3 hours, proportionate amounts of the solvents are used.
Yield: 73 mg (28%)
$^1$H-NMR (400 MHz, $CD_3OD$): δ = 1,22 (t, 3 H), 1,57-2,19 (m, 8 H), 2,92 (q, 2 H), 3,69 (quint, 1 H), 7,58-7,63 (t, 1 H), 7,72-7,79 (t, 1 H), 7,92 (d, 1 H), 8,15 (d, 1 H), 8,29 (d, 1 H), 8,40 (d, 1 H) ppm.

**Example 8**

7-Cyclopentyl-5-ethyl-2-(4-pyridyl)imidazo [5,1-f]triazin-4(3H)-one

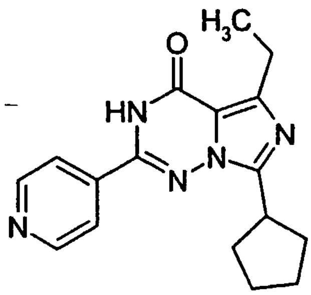

In analogy to the procedure for Example 1, 25 mg (0,37 mmol) of Example 21A, 56 mg (0,37 mmol) phosphoric trichloride are stirred at reflux for 2 hours, proportionate amounts of the solvents are used.
Yield: 125 mg (100%)
LC/MS (A): MS (ESI): 310 (M+H$^+$), retention time 3.00 min.

**Example 9**

7-Cyclopentyl-2-(4,6-dimethyl-2-pyridinyl)-5-ethylimidazo[5,1-f][1,2,4]triazin-4(3H)-one

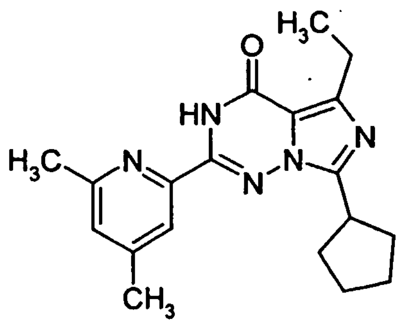

In analogy to the procedure for Example 1, 2,25 g (6,33 mmol) of Example 22A, 971 mg (6,33 mmol) phosphoric trichloride are stirred at reflux for 2 hours, proportionate amounts of the solvents are used.
Yield: 120 mg (6%)
LC/MS (A): MS (ESI): 337 ($M+H^+$), retention time 4.30 min.

**Example 10**

7-Cyclopentyl-5-ethyl-2-(3-pyridinyl)imidazo[5,1-f][1,2,4]triazin-4(3H)-one

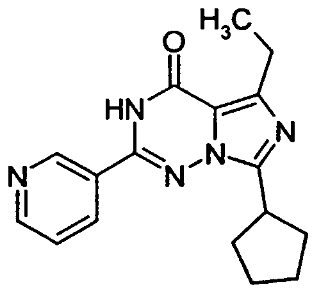

In analogy to the procedure for Example 1, 2,25 g (6,33 mmol) of Example 23A, 971 mg (6,33 mmol) phosphoric trichloride are stirred at reflux for 2 hours, proportionate amounts of the solvents are used.
Yield: 120 mg (6%)
$^1$H-NMR (300 MHz, DMSO): $\delta$ = 12.00 (br. s, 1H), 9.10 (d, J=2Hz, 1H), 8.75 (m, 1H), 8.30 (m, 1H), 7.60 (dd, J=5Hz, J=7Hz, 1H), 3.60 (m, 1H), 2.90 (q, J=7Hz, 2H), 2.20-1.60 (m, 8H), 1.25 (t, J=7Hz, 3H) ppm.

**Example 11**

7-Cyclopentyl-5-ethyl-2-(2-thienyl)imidazo[5,1-f][1,2,4]triazin-4(3H)-one

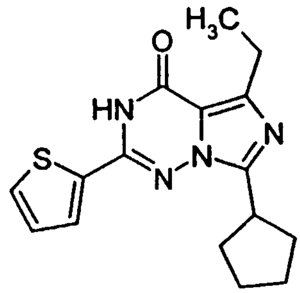

In analogy to the procedure for Example 1, 500 mg (1,50 mmol) of Example 24A, 384 mg (1,50 mmol) phosphoric trichloride are stirred at reflux for 2 hours, proportionate amounts of the solvents are used.
Yield: 390 mg (82%)
$^1$H-NMR (300 MHz, DMSO): δ = 12.10 (br. s, 1H), 8.10 (d, J=3Hz, 1H), 7.85 (d, J=5Hz, 1H), 7.20 (dd, J=3Hz, J=5Hz, 1H), 3.50 (m, 1H), 2.90 (q, J=7Hz, 2H), 2.20-1.60 (m, 8H), 1.20 (t, J=7Hz, 3H) ppm.

**Example 12**

7-Cyclopentyl-5-ethyl-2-(2-pyridinyl)imidazo[5,1-f][1,2,4]triazin-4(3H)-one

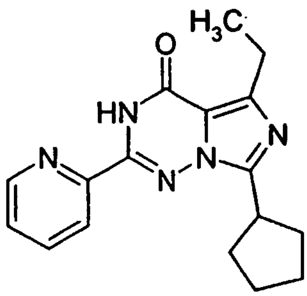

In analogy to the procedure for Example 1, 940 mg (2,87 mmol) of Example 25A, 440 mg (2,87 mmol) phosphoric trichloride are stirred at reflux for 4 hours, proportionate amounts of the solvents are used.
Yield: 440 mg (49%)
$^1$H-NMR (300 MHz, DMSO): δ = 11.20 (br. s, 1H), 8.80 (d, J=2Hz, 1H), 8.25 (d, J=7Hz, 1H), 8.05 (m, 1H), 7.65 (m, 1H), 3.60 (m, 1H), 2.90 (q, J=7Hz, 2H), 2.20-1.60 (m, 8H), 1.20 (t, J=7Hz, 3H) ppm.

**Example 13**

7-Cyclopentyl-5-ethyl-2-(2-furyl)imidazo[5,1-f][1,2,4]triazin-4(3H)-one

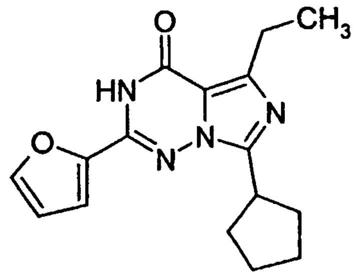

In analogy to the procedure for Example 1, 380 mg (1,20 mmol) of Example 26A, 184 mg (1,20 mmol) phosphoric trichloride are stirred at reflux for 4 hours, proportionate amounts of the solvents are used.
Yield: 100 mg (28%)
$^1$H-NMR (300 MHz, DMSO): δ = 12.00 (br. s, 1H), 8.00 (m, 1H), 7.55 (m, 1H), 6.75 (m, 1H), 3.50 (m, 1H), 2.85 (q, J=7Hz, 2H), 2.20-1.60 (m, 8H), 1.20 (t, J=7Hz, 3H) ppm.

**Example 14**

7-(cis-4-tert-Butylcyclohexyl)-5-ethyl-2-(2-thienyl)imidazo[5,1-f][1,2,4]triazin-4(3H)-one

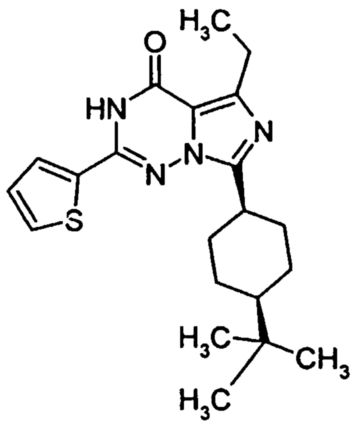

1,6 g (3,98 mmol, 1 equiv.) of Example 27A are suspended in 28 ml dichloroethane, and 2,27 g (14,8 mmol, 4 equiv.) phosphoroxychloride are added. The mixture is stirred at reflux for 4 hours. After cooling down to room temperature, dichloromethane is added and the organic phase is quenched with water, washed with water, dried over magnesium sulfate and evaporated to dryness *in vacuo.* The solid residue is washed with diethyl ether, filtered and dried.
Yield: 0.67 g (45%)
$^1$H-NMR (300 MHz, DMSO): δ = 0.83 (s, 9H); 1.01-1.13 (m, 1H); 1.18 (t, 3H); 1.49-1.75 (m, 6H); 2.20 (m, 2H), 2.88 (q, 2H); 3.47 (m, 1H); 7.20 (dd, 1H); 7.80 (dd, 1H); 8.08 (dd, 1H); 11.92 (s, 1H) ppm.

**Example 15**

7-Cyclobutyl-5-ethyl-2-(2-thienyl)imidazo[5,1-f][1,2,4]triazin-4(3H)-one

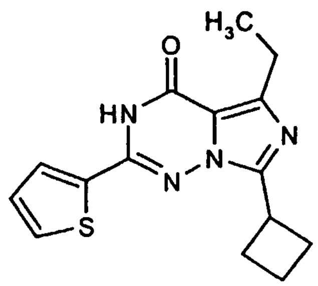

In analogy to the procedure for Example 1, 140 mg (0,44 mmol) of Example 28A, 165 mg (1,07 mmol) phosphoric trichloride are stirred at reflux for 3 hours, proportionate amounts of the solvents are used.
Yield: 31 mg (23%)
$^1$H-NMR (300 MHz, DMSO): δ = 1.24 (t, 3H); 1.88-2.52 (t, 6H); 2.88 (q, 2H); 3.93 (m, 1H); 7.22 (m, 1H); 7.84 (dd, 1H); 8.08 (dd, 1H); 12.01 (s, 1H) ppm.

**Example 16**

7-(trans-4-tert-Butylcyclohexyl)-5-ethyl-2-(2-thienyl)imidazo[5,1-f][1,2,4]triazin-4(3H)-one

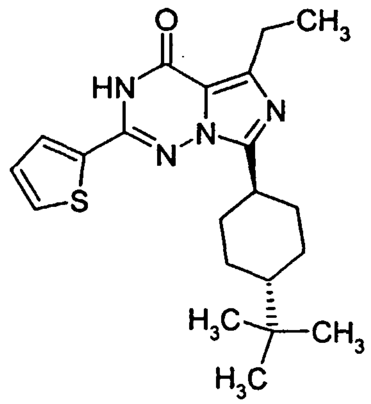

In analogy to the procedure for Example 1, 580 mg (1,44 mmol) of Example 29A, 820 mg (5,36 mmol) phosphoric trichloride are stirred at reflux for 4 hours, proportionate amounts of the solvents are used.
Yield: 85 mg (15%)
$^1$H-NMR (300 MHz, DMSO): δ = 0.89 (s, 9H); 1.12 (m, 2H); 1.22 (m, 4H); 1.62 (m, 2H); 1.87 (m, 2H); 2.03 (m, 2H); 2.87 (q, 2H); 2.86-3.07 (m, 1H); 7.21 (dd, 1H); 7.82 (dd, 1H); 8.08 (dd, 1H); 11.97 (s, 1H) ppm.

**Example 17**

5-Ethyl-7-isobutyl-2-(2-thienyl)imidazo[5,1-f][1,2,4]triazin-4(3H)-one

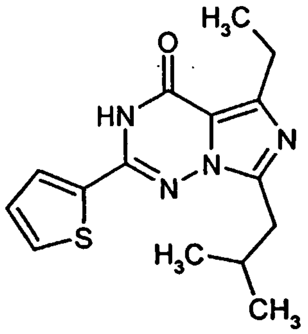

In analogy to the procedure for Example 1, 270 mg (0,84 mmol) of Example 30A, 235 mg (1.53 mmol) phosphoric trichloride are stirred at reflux for 4 hours, proportionate amounts of the solvents are used.
Yield: 4.5 mg (2%)
$^1$H-NMR (300 MHz, DMSO): δ = 0.94 (d, 6H); 1.23 (t, 3H); 2.17 (m, 1H); 2.79-2.97 (m, 4H); 7.21 (dd, 1H); 7.82 (dd, 1H); 8.10 (dd, 1H); 12.00 (s, 1H) ppm.

**Example 18**

2-(5-Chloro-2-thienyl)-7-cyclopentyl-5-ethylimidazo[5,1-f][1,2,4]triazin-4(3H)-one

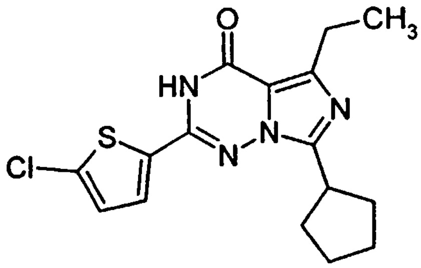

In analogy to the procedure for Example 1, 203 mg (0,55 mmol) crude N-{1-[3-(5-chloro-2-thienyl)-5-oxo-4,5-dihydro-1,2,4-triazin-6-yl]propyl}cyclopentanecarboxamide, 127 mg (0,83 mmol) phosphoric trichloride are stirred at reflux for 3 hours, proportionate amounts of the solvents are used.
Yield: 67 mg (35%)
$^1$H-NMR (400 MHz, $CD_3OD$): δ = 1,28 (t, 3 H), 1,56-2,18 (m, 8 H), 2,96 (q, 2 H), 3,60 (quint, 1 H), 7,09 (d, 1 H), 7,72 (d, I H) ppm.

**Example 19**

cis-7-(4-tert-Butylcyclohexyl)-2-(5-chloro-2-thienyl)-5-ethylimidazo[5,1-f][1,2,4]-triazin-4(3H)-one

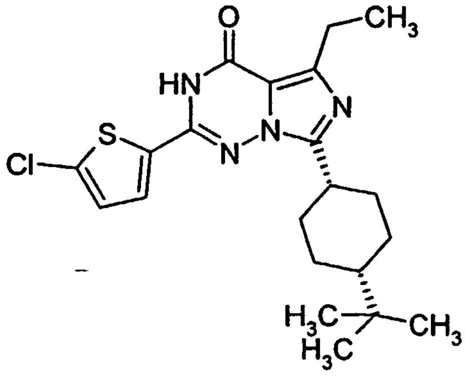

In analogy to the procedure for Example 1, 322 mg (0,74 mmol) crude cis-4-tert-butyl-N-{1-[3-(5-chloro-2-thienyl)-5-oxo-4,5-dihydro-1,2,4-triazin-6-yl]propyl}-cyclohexanecarboxamide, 169 mg (1,10 mmol) phosphoric trichloride are stirred at reflux for 3 hours, proportionate amounts of the solvents are used.
Yield: 72 mg (23%)
$^1$H-NMR (400 MHz, $CD_3OD$): δ = 0,85 (s, 9 H), 0,96-2,40 (m, 12 H, t at 1,27), 2,96 (q, 2 H), 3,48 (m, 1 H), 7,11 (d, 1 H), 7,79 (d, 1 H) ppm.

**Example 20**

7-Cyclopentyl-5-ethyl-2-(1-isoquinolinyl)imidazo[5,1-f][1,2,4]triazin-4(3H)-one

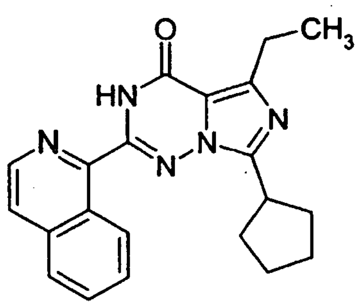

In analogy to the procedure for Example 1, 402 mg (1,07 mmol) crude N-{1-[3-(1-isoquinolinyl)-5-oxo-4,5-dihydro-1,2,4-triazin-6-yl]propyl}cyclopentanecarboxamide, 245 mg (1,60 mmol) phosphoric trichloride are stirred at reflux for 3 hours, proportionate amounts of the solvents are used.
Yield: 115 mg (30%)
$^1$H-NMR (400 MHz, $CD_3OD$): δ = 1,32 (t, 3 H), 1,55-2,24 (m, 8 H), 3,02 (q, 2 H), 3,71 (quint, 1 H), 7,79 (t, 1 H), 7,86 (t, 1 H), 8,02 (d, 1 H), 8,07 (d, 1 H), 8,66 (d, 1 H), 9,15 (d, 1 H) ppm.

**Example 21**

cis-7-(4-tert-Butylcyclohexyl)-5-ethyl-2-(1-isoquinolinyl)imidazo[5,1-f][1,2,4]-triazin-4(3H)-one

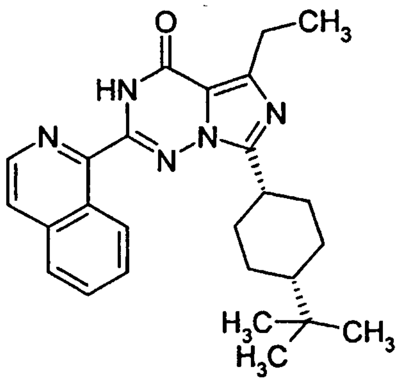

In analogy to the procedure for Example 1, 318 mg (0,71 mmol) crude cis-4-tert-butyl-N-{1-[3-(1-isoquinolinyl)-5-oxo-4,5-dihydro-1,2,4-triazin-6-yl]propyl}cyclohexanecarboxamide, 164 mg (1,07 mmol) phosphoric trichloride are stirred at reflux for 3 hours, proportionate amounts of the solvents are used.
Yield: 111 mg (36%)
$^1$H-NMR (400 MHz, $CD_3OD$): δ = 0,85 (s, 9 H), 1,01-2,48 (m, 12 H, t at 1,33), 3,04 (q, 2 H), 3,65 (m, 1 H), 7,78 (t, I H), 7,85 (t, 1 H), 8,01 (d, 1 H), 8,06 (d, 1 H), 8,64 (d, 1 H), 9,21 (d, 1 H) ppm.

**Example 22**

2-(3-Bromo-2-thienyl)-7-cyclopentyl-5-ethylimidazo[5,1-f][1,2,4]triazin-4(3H)-one

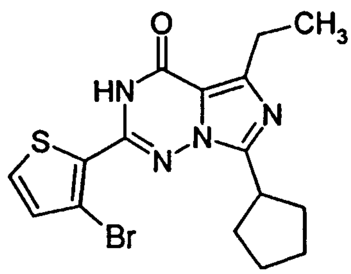

In analogy to the procedure for Example 1, 400 mg (0,97 mmol) crude N-{1-[3-(3-bromo-2-thienyl)-5-oxo-4,5-dihydro-1,2,4-triazin-6-yl]propyl}cyclopentanecarboxamide, 298 mg (1,95 mmol) phosphoric trichloride are stirred at reflux for 3 hours, proportionate amounts of the solvents are used.
Yield: 340 mg (89%)
$^1$H-NMR (400 MHz, $CDCl_3$): δ = 1,32 (t, 3 H), 1,66-2,19 (m, 8 H), 3,01 (q, 2 H), 3,57 (quin., 1 H), 7,11 (d, 1 H), 7,49 (d, 1 H) ppm.

**Example 23**

cis-2-(3-Bromo-2-thienyl)-7-(4-tert-butylcyclohexyl)-5-ethylimidazo[5,1-f][1,2,4]-triazin-4(3H)-one

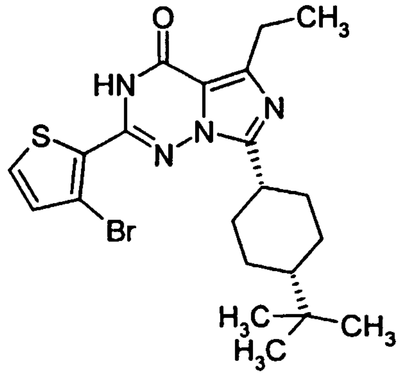

In analogy to the procedure for Example 1, 611 mg (1,27 mmol) crude cis-N-{1-[3-(3-bromo-2-thienyl)-5-oxo-4,5-dihydro-1,2,4-triazin-6-yl]propyl}-4-tert-butylcyclohexanecarboxamide, 389 mg (2,54 mmol) phosphoric trichloride are stirred at reflux for 3 hours, proportionate amounts of the solvents are used.
Yield: 275 mg (47%)
$^1$H-NMR (400 MHz, $CD_3OD$): $\delta$ = 0,85 (s, 9 H), 1,07-2,42 (m, 12 H, t at 1,29), 2,99 (q, 2 H), 3,50 (m, 1 H), 7,18 (d, 1 H), 7,73 (d, 1 H) ppm.

**Example 24**

trans-2-(3-Bromo-2-thienyl)-7-(4-tert-butylcyclohexyl)-5-ethylimidazo[5,1-f][1,2,4]-triazin-4(3H)-one

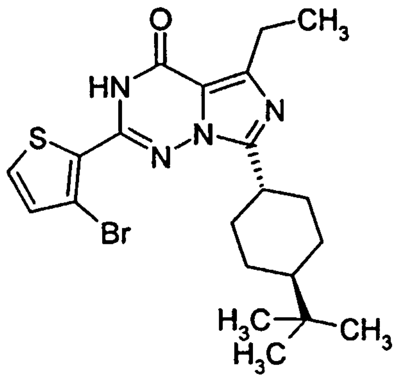

In analogy to the procedure for Example 1, 306 mg (0,64 mmol) crude trans-N-{1-[3-(3-bromo-2-thienyl)-5-oxo-4,5-dihydro-1,2,4-triazin-6-yl]propyl}-4-tert-butylcyclohexanecarboxamide, 292 mg (1,91 mmol) phosphoric trichloride are stirred at reflux for 3 hours, proportionate amounts of the solvents are used.
Yield: 194 mg (66%)
$^1$H-NMR (400 MHz, $CD_3OD$): $\delta$ = 0,90 (s, 9 H), 0,99-1,41 (m, 6 H, t at 1,29), 1,69-2,10 (m, 6 H), 2,97 (q, 2 H), 3,17 (m, 1 H), 7,21 (d, 1 H), 7,76 (d, 1 H) ppm.

**Example 25**

cis/trans-2-(5-Chloro-2-thienyl)-5-ethyl-7-(4-methylcyclohexyl)imidazo[5,1-f]-[1,2,4]triazin-4(3H)-one

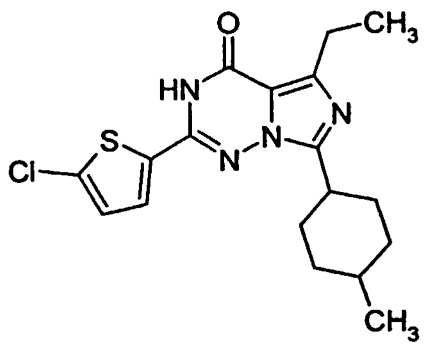

In analogy to the procedure for Example 1, 731 mg (1,85 mmol) crude N-{1-[3-(5-chloro-2-thienyl)-5-oxo-4,5-dihydro-1,2,4-triazin-6-yl]propyl}-4-methylcyclohexanecarboxamide, 851 mg (5,55 mmol) phosphoric trichloride are stirred at reflux for 3 hours, proportionate amounts of the solvents are used.
Yield: 314 mg (45%)
$^1$H-NMR (300 MHz, $CD_3OD$): δ = 0,87-0,92 (m, 3 H), 1,05-2,20 (m, 12 H, t at 1,26 and 1,27), 2,90-3,00 (m, 2 H), 3,34-3,38 (m, 1 H), 7,08 (d, 1 H), 7,69 (d, 1 H) ppm.

**Example 26**

7-Cyclobutyl-5-ethyl-2-(2-pyrazinyl)imidazo[5,1-f][1,2,4]triazin-4(3H)-one

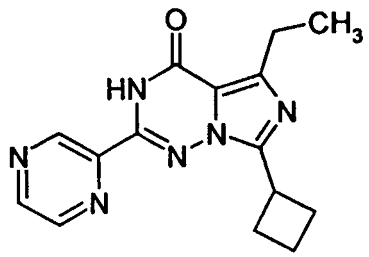

200 mg (0.86 mmol, 1 equiv.) of Example 60A are suspended in 10 ml dichloroethane, and 130 mg (1.29 mmol) triethylamine and 102 mg (0.86 mmol) cyclobutanecarbonyl chloride are added. The mixture is stirred at room temperature for one hour, then 198 mg (1.29 mmol) phosphoroxychloride are added. The mixture is stirred at reflux for 3 hours. After cooling down to room temperature, ethyl acetate and saturated $NaHCO_3$ (aq) are added. The organic phase is washed with saturated $NaHCO_3$ (aq), water and brine, dried over sodium sulfate and evaporated to dryness *in vacuo.* The product is purified by chromatography (flash or column chromatography or preparative HPLC).
Yield: 35 mg (14%)
LC/MS (A): MS (ES+): 297 ($M+H^+$), retention time 2.04 min.

**Example 27**

7-Cyclopentyl-5-ethyl-2-(2-pyrazinyl)imidazo[5,1-f][1,2,4]triazin-4(3H)-one

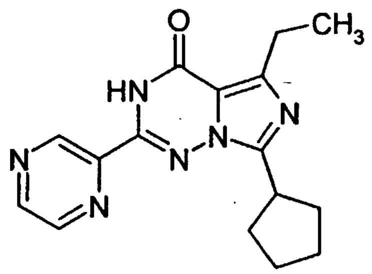

In analogy to the procedure for Example 26, 200 mg (0,86 mmol) of Example 60A, 114 mg (0.86 mmol) cyclopentanecarbonyl chloride and proportionate amounts of the other reagents are used.
Yield: 88 mg (33%)
$^1$H-NMR (DMSO-$d_6$, 200 MHz): $\delta$ = 1.2 (t, 3H), 1.5-2.1 (m, 8H), 2.9 (q, 2H), 3.6 (m, 1H), 8.8 (m, 1H), 8.9 (m, 1H), 9.4 (m, 1H), 11.6 (br.s, 1H) ppm.

**Example 28 and Example 29**

7-(4-tert-Butylcyclohexyl)-5-ethyl-2-(2-pyrazinyl)imidazo[5,1-f][1,2,4]triazin-4(3H)-one

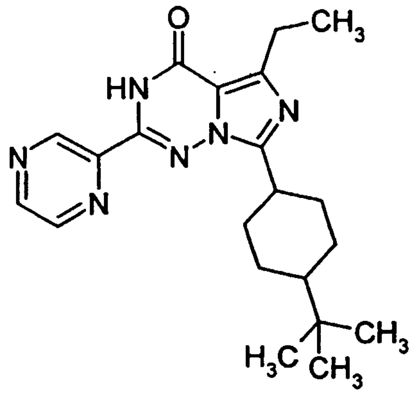

In analogy to the procedure for Example 26, 500 mg (2.15 mmol) of Example 60A, 436 mg (2.15 mmol) 4-tert-butylcyclohexanecarbonyl chloride and proportionate amounts of the other reagents are used.
Yield: 177 mg (23%) cis-isomer
28 mg (3%) trans-isomer
cis-isomer (Example 28):
$^1$H-NMR (DMSO-$d_6$, 200 MHz): $\delta$ = 0.9 (s, 9H), 1.1 (m, 1H), 1.2 (t, 3H), 1.5-1.8 (m, 6H), 2.2 (m, 2H), 2.9 (q, 2H), 3.6 (m, 1H), 8.8 (m, 1H), 8.9 (m, 1H), 9.3 (m, 1H), 11.7 (br. s, 1H) ppm.
trans-isomer (Example 29):
$^1$H-NMR (DMSO-$d_6$, 200 MHz): $\delta$ = 0.9 (s, 9H), 1.2 (t, 3H), 1.2 (m, 3H), 1.6 (m, 2H), 1.8 (m, 2H), 2.0 (m, 2H), 2.9 (q, 2H), 3.2 (m, 1H), 8.8 (m, 1H), 8.9 (m, 1H), 9.4 (m, 1H), 11.6 (br. s, 1H) ppm.

**Example 30**

7-Cyclopentyl-5-ethyl-2-(2-methyl-1,3-thiazol-4-yl)imidazo[5,1-f][1,2,4]triazin-4(3H)-one

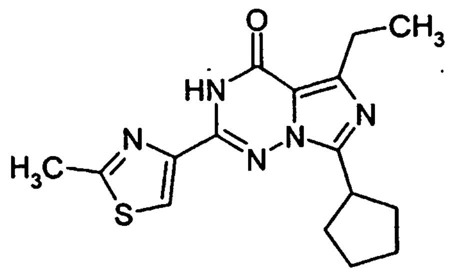

In analogy to the procedure for Example 26, 200 mg (0.60 mmol) of Example 61A, 79 mg (0.60 mmol) cyclopentanecarbonyl chloride and proportionate amounts of the other reagents are used.
Yield: 117 mg (60%)
$^1$H-NMR (DMSO-$d_6$, 200 MHz): $\delta$ = 1.2 (t, 3H), 1.5-1.9 (m, 6H), 2.2 (m, 2H), 2.7 (s, 3H), 2.9 (q, 2H), 3.6 (m, 1H), 8.4 (s, 1H), 11.4 (br. s, 1H) ppm.

**Example 31**

cis-7-(4-tert-Butylcyclohexyl)-5-ethyl-2-(2-methyl-1,3-thiazol-4-yl)imidazo[5,1-f]-[1,2,4] triazin-4(3 H)-one

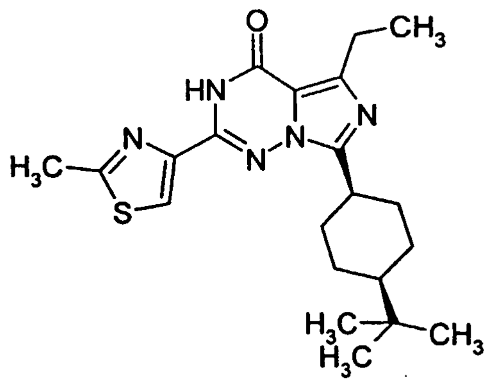

In analogy to the procedure for Example 26, 250 mg (0.99 mmol) of Example 61A, 202 mg (0.99 mmol) 4-tert-butylcyclohexanecarbonyl chloride and proportionate amounts of the other reagents are used.
Yield: 98 mg (25%) cis-isomer
$^1$H-NMR (DMSO-$d_6$, 200 MHz): $\delta$ = 0.8 (s, 9H), 1.1 (m, 1H), 1.2 (t, 3H), 1.5-1.7 (m, 6H), 2.2 (m, 2H), 2.7 (s, 3H), 2.9 (q, 2H), 3.5 (m, 1H), 8.3 (s, 1H), 11.4 (br. s, 1H) ppm.

**Example 32**

cis-7-(4-tert-Butylcyclohexyl)-5-ethyl-2-(1,3-thiazol-2-yl)imidazo[5,1-f][1,2,4]-triazin-4(3H)-one

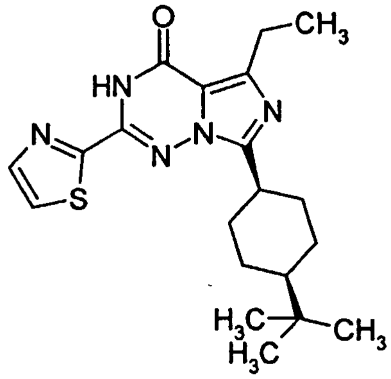

In analogy to the procedure for Example 26, 250 mg (1.05 mmol) of Example 62A, 214 mg (1.05 mmol) 4-tert-butylcyclohexanecarbonyl chloride and proportionate amounts of the other reagents are used.
Yield: 86 mg (21%) cis-isomer
$^1$H-NMR (DMSO-$d_6$, 200 MHz): δ = 0.8 (s, 9H), 1.1 (m, 1H), 1.2 (t, 3H), 1.5-1.7 (m, 6H), 2.2 (m, 2H), 2.9 (q, 2H), 3.5 (m, 1H), 8.1 (m, 2H), 11.9 (br. s, 1H) ppm.

**Example 33**

7-Cyclopentyl-5-ethyl-2-(1,3-thiazol-2-yl)imidazo[5,1-f][1,2,4]triazin-4(3H)-one

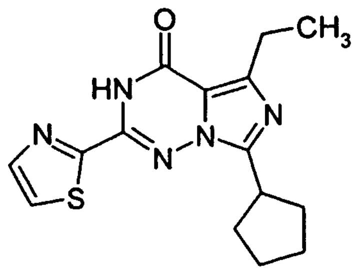

In analogy to the procedure for Example 26, 150 mg (0.63 mmol) of Example 62A, 84 mg (0.63 mmol) cyclopentanecarbonyl chloride and proportionate amounts of the other reagents are used.
Yield: 73 mg (37%)
$^1$H-NMR (DMSO-$d_6$, 200 MHz): δ = 1.2 (t, 3H), 1.5-1.9 (m, 6H), 2.1 (m, 2H), 2.9 (q, 2H), 3.5 (m, 1H), 8.1 (m, 2H), 11.9 (br. s, 1H) ppm.

**Example 34**

7-Cyclopentyl-2-(3,5-difluoro-2-pyridinyl)-5-ethylimidazo[5,1-f][1,2,4]triazin-4(3H)-one

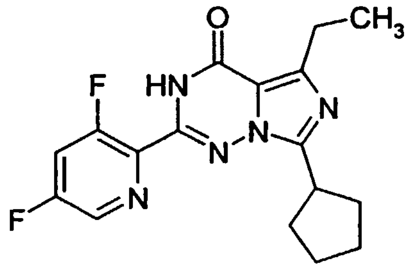

In analogy to the procedure for Example 26, 300 mg (1.12 mmol) of Example 63A, 223 mg (1.68 mmol) cyclopentanecarbonyl chloride and proportionate amounts of the other reagents are used.
Yield: 25 mg (6%)
LC/MS (A): MS (ES+): 346 ($M+H^+$), retention time 2.52 min.

**Example 35 and Example 36**

7-(4-tert-Butylcyclohexyl)-2-(3,5-difluoro-2-pyridinyl)-5-ethylimidazo[5,1-f][1,2,4]-triazin-4(3H)-one

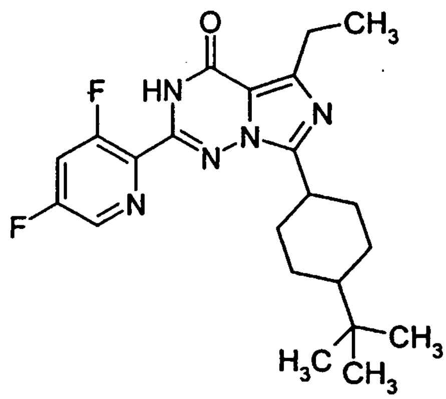

In analogy to the procedure for Example 26, 500 mg (1.87 mmol) of Example 63A, 569 mg (2.81 mmol) 4-tert-butylcyclohexanecarbonyl chloride and proportionate amounts of the other reagents are used.
Yield: 4 mg (1%) cis-isomer
17.4 mg (3%) trans-isomer
cis-isomer (Example 35):
LC/MS (A): MS (ES+): 416 ($M+H^+$), retention time 3.20 min.
trans-isomer (Example 36):
$^1$H-NMR (DMSO-$d_6$, 300 MHz): δ = 0.9 (s, 9H), 1.1 (m, 3H), 1.2 (t, 3H), 1.6 (m, 2H), 1.8 (m, 2H), 2.0 (m, 2H), 2.9 (q, 2H), 3.0 (m, 1H), 8.2 (m, 1H), 8.7 (m, 1H), 11.6 (br. s, 1H) ppm.

**Example 37**

cis-7-(4-tert-Butylcyclohexyl)-2-(1,5-dimethyl-1H-pyrrol-2-yl)-5-ethylimidazo[5,1-f][1,2,4]triazin-4(3H)-one

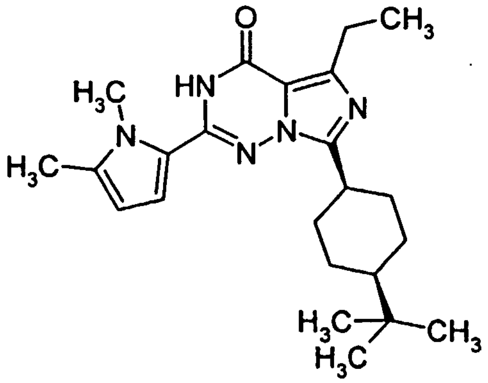

In analogy to the procedure for Example 26, 250 mg (1.01 mmol) of Example 64A, 205 mg (1.01 mmol) 4-tert-butylcyclohexanecarbonyl chloride and proportionate amounts of the other reagents are used.
Yield: 65 mg (21%) cis-isomer
$^1$H-NMR (DMSO-$d_6$, 400 MHz): $\delta$ = 0.8 (s, 9H), 1.1 (m, 1H), 1.2 (t, 3H), 1.5-1.7 (m, 6H), 2.2 (m, 2H), 2.3 (s, 3H), 2.9.(q, 2H), 3.5 (m, 1H), 3.8 (s, 3H), 6.0 (d, 1H), 7.0 (d, 1H), 12.0 (br. s, 1H) ppm.

**Example 38**

7-Cyclopentyl-2-(1,5-dimethyl-1H-pyrrol-2-yl)-5-ethylimidazo[5,1-f][1,2,4]triazin-4(3H)-one

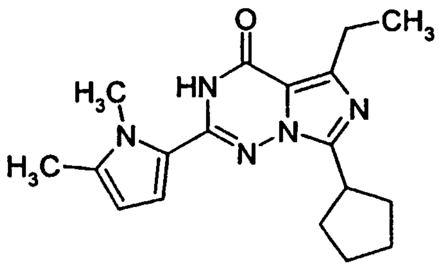

In analogy to the procedure for Example 26, 150 mg (0.61 mmol) of Example 64A, 80 mg (0.61 mmol) cyclopentanecarbonyl chloride and proportionate amounts of the other reagents are used.
Yield: 65 mg (33%)
$^1$H-NMR (DMSO-$d_6$, 200 MHz): $\delta$ = 1.2 (t, 3H), 1.5-2.0 (m, 8H), 2.2 (s, 3H), 2.9 (q, 2H), 3.5 (m, 1H), 3.8 (s, 3H), 6.0 (d, 1H), 7.0 (d, 1H), 11.3 (br. s, 1H) ppm.

**Example 39**

2-(3-Bromo-2-pyridinyl)-7-cyclopentyl-5-ethylimidazo[5,1-f][1,2,4]triazin-4(3H)-one

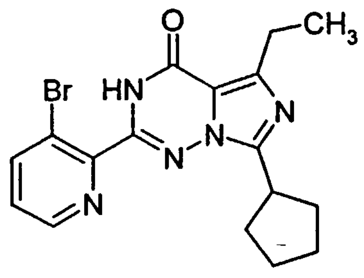

In analogy to the procedure for Example 26, 100 mg (0.32 mmol) of Example 65A, 43 mg (0.32 mmol) cyclopentanecarbonyl chloride and proportionate amounts of the other reagents are used.
Yield: 30 mg (24%)
$^1$H-NMR (DMSO-$d_6$, 200 MHz): $\delta$ = 1.2 (t, 3H), 1.6 (m, 2H), 1.7 (m, 2H), 1.9 (m, 2H), 2.0 (m, 2H), 2.9 (q, 2H), 3.5 (m, 1H), 7.6 (d/d, 1H), 8.3 (d/d, 1H), 8.7 (d/d, 1H), 11.9 (s, 1H) ppm.

**Example 40**

cis-2-(3-Bromo-2-pyridinyl)-7-(4-tert-butylcyclohexyl)-5-ethylimidazo[5,1-f]-[1,2,4]triazin-4(3H)-one

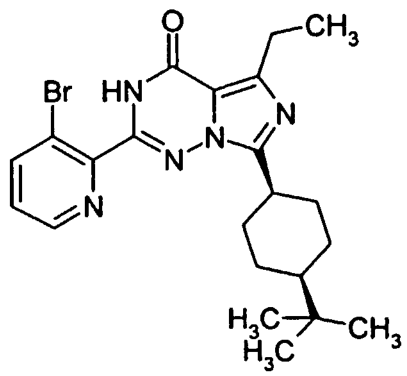

In analogy to the procedure for Example 26, 110 mg (0.35 mmol) of Example 65A, 72 mg (0.35 mmol) 4-cis-tert-butylcyclohexanecarbonyl chloride and proportionate amounts of the other reagents are used.
Yield: 92 mg (56%)
$^1$H-NMR (DMSO-$d_6$, 200 MHz): $\delta$ = 0.8 (s, 9H), 1.1 (m, 1H), 1.2 (t, 3H), 1.5-1.7 (m, 6H), 2.2 (m, 2H), 2.9 (q, 2H), 3.4 (m, 1H), 7.6 (d/d, 1H), 8.3 (d/d, 1H), 8.7 (d/d, 1H), 12.0 (s, 1H) ppm.

**Example 41**

cis-7-(4-tert-Butylcyclohexyl)-2-(3-chloro-2-pyridinyl)-5-ethylimidazo[5,1-f][1,2,4]-triazin-4(3H)-one

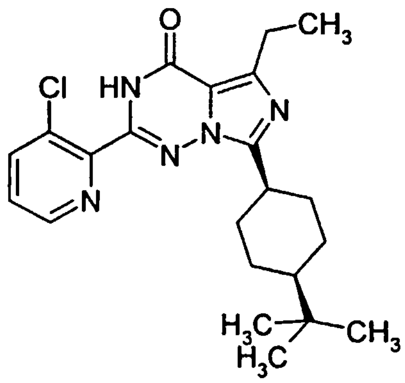

In analogy to the procedure for Example 26, 150 mg (0.56 mmol) of Example 66A, 114 mg (0.56 mmol) 4-cis-tert-butylcyclohexanecarbonyl chloride and proportionate amounts of the other reagents are used.
Yield: 106 mg (45%)
$^1$H-NMR (DMSO-$d_6$, 300 MHz): $\delta$ = 0.8 (s, 9H), 1.1 (m, 1 H), 1.2 (t, 3H), 1.5-1.7 (m, 6H), 2.2 (m, 2H), 2.9 (q, 2H), 3.4 (m, 1H), 7.7 (d/d, 1H), 8.2 (d/d, 1H), 8.7 (d/d, 1H), 11.9 (s, 1H) ppm.

**Example 42**

2-(3-Chloro-2-pyridinyl)-7-cyclopentyl-5-ethylimidazo[5,1-f][1,2,4]triazin-4(3H)-one

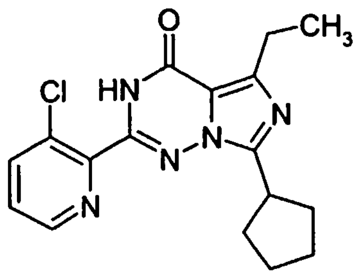

In analogy to the procedure for Example 26, 150 mg (0.56 mmol) of Example 66A, 75 mg (0.56 mmol) cyclopentanecarbonyl chloride and proportionate amounts of the other reagents are used.
Yield: 119 mg (61%)
$^1$H-NMR (DMSO-$d_6$, 200 MHz): $\delta$ = 1.2 (t, 3H), 1.5-2.0 (m, 8H), 2.9 (q, 2H), 3.4 (m, 1H), 7.7 (d/d, 1H), 8.2 (d/d, 1H), 8.7 (d/d, 1H), 11.9 (s, 1H) ppm.

**Example 43**

7-Cyclopentyl-5-ethyl-2-(1H-pyrrol-2-yl)imidazo[5,1-f][1,2,4]triazin-4(3H)-one

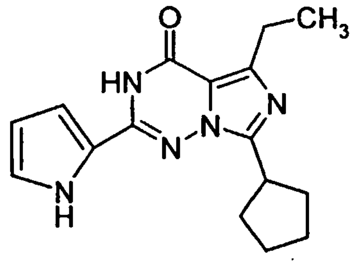

In analogy to the procedure for Example 26, 150 mg (0.68 mmol) of Example 67A, 91 mg (0.68 mmol) cyclopentanecarbonyl chloride and proportionate amounts of the other reagents are used.
Yield: 100 mg (49%)
$^1$H-NMR (DMSO-$d_6$, 200 MHz): δ = 1.2 (t, 3H), 1.7 (m, 2H), 1.8 (m, 4H), 2.1 (m, 2H), 2.9 (q, 2H), 3.6 (m, 1H), 6.2 (m, 1H), 7.0 (m, 1H), 7.2 (m, 1H), 11.4 (s, 1H), 11.5 (br. s, 1H) ppm.

**Example 44**

7-Cyclopentyl-5-ethyl-2-(3-furyl)imidazo[5,1-f][1,2,4]triazin-4(3H)-one

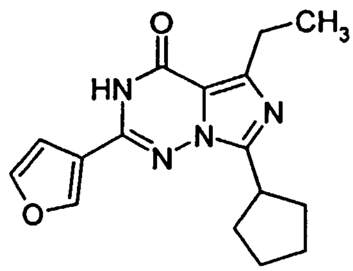

In analogy to the procedure for Example 26, 250 mg (1.14 mmol) of Example 68A, 150 mg (1.14 mmol) cyclopentanecarbonyl chloride and proportionate amounts of the other reagents are used.
Yield: 48 mg (14%)
$^1$H-NMR (DMSO-$d_6$, 200 MHz): δ = 1.2 (t, 3H), 1.7 (m, 2H), 1.8 (m, 4H), 2.1 (m, 2H), 2.9 (q, 2H), 3.6 (m, 1H), 7.0 (m, 1H), 7.9 (m, 1H), 8.5 (m, 1H), 11.7 (s, 1H) ppm.

**Example 45**

cis-7-(4-tert-Butylcyclohexyl)-5-ethyl-2-(3-furyl)imidazo[5,1-f][1,2,4]triazin-4(3H)-one

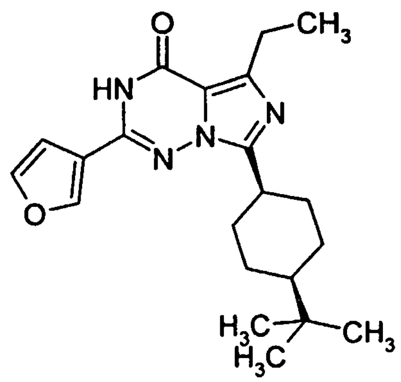

In analogy to the procedure for Example 26, 500 mg (2.27 mmol) of Example 68A, 460 mg (2.27 mmol) 4-cis-tert-butylcyclohexanecarbonyl chloride and proportionate amounts of the other reagents are used.
Yield: 101 l mg (12%)
$^1$H-NMR (DMSO-$d_6$, 300 MHz): δ = 0.8 (s, 9H), 1.1 (m, 1H), 1.2 (t, 3H), 1.6 (m, 2H), 1.8 (m, 4H), 2.2 (m, 2H), 2.9 (q, 2H), 3.6 (m, 1H), 7.0 (m, 1H), 7.9 (m, 1H), 8.5 (m, 1H), 11.7 (br. s, 1H), 11.9 (s, 1H) ppm.

**Example 46**

cis-7-(4-tert-Butylcyclohexyl)-5-ethyl-2-(1-methyl-1H-pyrrol-2-yl)imidazo[5,1-f]-[1,2,4]triazin-4(3H)-one

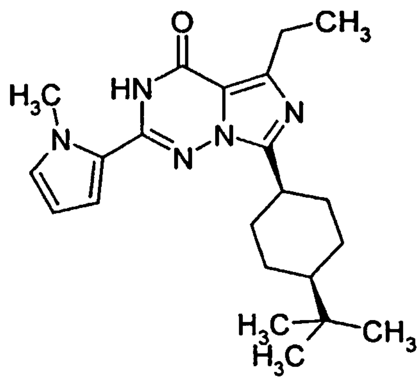

In analogy to the procedure for Example 26, 1000 mg (4.29 mmol) of Example 69A, 434 mg (2.14 mmol) 4-cis-tert-butylcyclohexanecarbonyl chloride and proportionate amounts of the other reagents are used.
Yield: 24 mg (2%)
$^1$H-NMR (DMSO-$d_6$, 300 MHz): δ = 0.8 (s, 9H), 1.1 (m, 1H), 1.2 (t, 3H), 1.6 (m, 6H), 2.2 (m, 2H), 2.9 (q, 2H), 3.5 (m, 1H), 3.9 (s, 3H), 6.1 (m, 1H), 7.1 (m, 2H), 11.4 (s, 1H)ppm.

**Example 47**

7-Cyclopentyl-5-ethyl-2-(1-methyl-1H-pyrrol-2-yl)imidazo[5,1-f][1,2,4]triazin-4(3H)-one

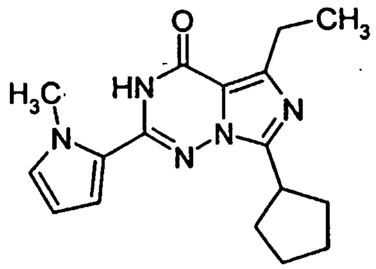

In analogy to the procedure for Example 26, 500 mg (2.14 mmol) of Example 69A, 142 mg (1.07 mmol) cyclopentanecarbonyl chloride and proportionate amounts of the other reagents are used.
Yield: 36 mg (5%)
$^1$H-NMR (DMSO-$d_6$, 200 MHz): $\delta$ = 1.2 (t, 3H), 1.6 (m, 2H), 1.7 (m, 2H), 1.9 (m, 2H), 2.0 (m, 2H), 2.9 (q, 2H), 3.5 (m, 1H), 3.9 (s, 3H), 6.1 (m, 1H), 7.1 (m, 2H), 11.3 (s, 1H) ppm.

**Example 48**

5-Ethyl-2-(2-thienyl)-7-[3-(trifluoromethyl)cyclohexyl]imidazo[5,1-f][1,2,4]triazin-4(3H)-one

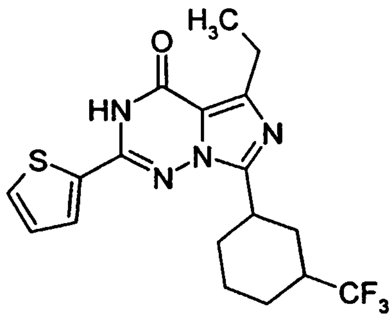

In analogy to the procedure for Example 1, 160 mg (0,39 mmol) of Example 75A, 165 mg (1,07 mmol) phosphoric trichloride are stirred at reflux for 3 hours, proportionate amounts of the solvents are used.
Yield: 11.9 mg (8%)
$^1$H-NMR (200 MHz, DMSO): $\delta$ = 1.20 (t, 3H); 1.50-2.20 (m, 8H); 2.60 (m, 1H); 2.90 (quart., 2H); 3.30 (m, 1H); 7.20 (m, 1H); 7.80 (m, 1H); 8.10 (m, 1H); 12.00 (s, 1H).

**Beispiel 49**

5-Ethyl-7-(4-methylcyclohexyl)-2-(2-thienyl)imidazo[5,1-f][1,2,4]triazin-4(3H)-one

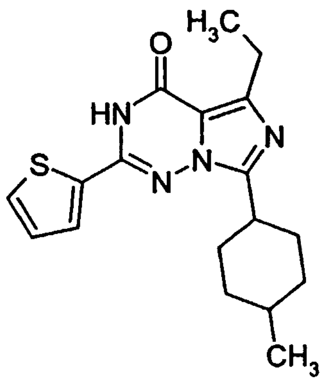

In analogy to the procedure for Example 1, 150 mg (0,42 mmol) of Example 76A, 165 mg (1,07 mmol) phosphoric trichloride are stirred at reflux for 3 hours, proportionate amounts of the solvents are used.
Yield: 21 mg (15%) of an isomeric mixture
$^1$H-NMR (200 MHz, DMSO): δ = 0.90-1.00 (2d, 3H); 1.20 (2t, 3H); 1.50-2.20 (m, 9H); 2.90 (2 quart., 2H); 3.20 (m, 1H); 7.20 (m, 1H); 7.80 (m, 1H); 8.10 (m, 1H); 12.00 (s, 1H).

**Example 50**

7-(Cyclohexylmethyl)-5-ethyl-2-(2-thienyl)imidazo[5,1-f][1,2,4]triazin-4(3H)-one

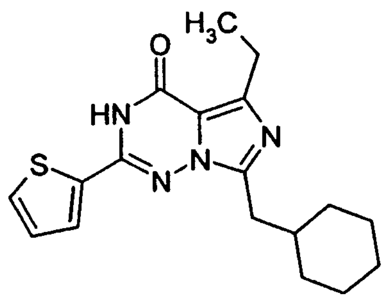

In analogy to the procedure for Example 1, 150 mg (0,42 mmol) of Example 77A, 165 mg (1,07 mmol) phosphoric trichloride are stirred at reflux for 3 hours, proportionate amounts of the solvents are used.
Yield: 100 mg (70%)
$^1$H-NMR (200 MHz, DMSO): δ = 0.90-1.30 (m, 9H); 1.60 (m, 4H), 1.85 (m, 1H); 2.90 (m, 4H); 7.20 (m, 1H); 7.80 (m, 1H); 8.10 (m, 1H); 12.00 (s, 1H).

**Example 51**

7-(1,4-Dimethylcyclohexyl)-5-ethyl-2-(2-thienyl)imidazo[5,1-f][1,2,4]triazin-4(3H)-one

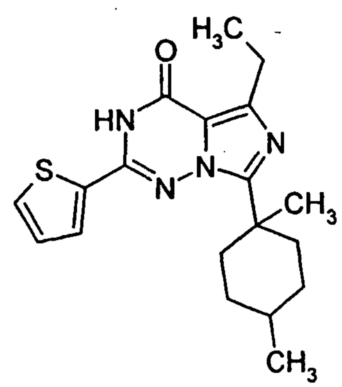

In analogy to the procedure for Example 1, 150 mg (0,40 mmol) of Example 78A, 165 mg (1,07 mmol) phosphoric trichloride are stirred at reflux for 3 hours, proportionate amounts of the solvents are used.
Yield: 90 mg (63%)
$^1$H-NMR (200 MHz, DMSO): δ = 0.70-2.10 (m, 18H); 2.91 (quart., 2H); 7.20 (m, 1H); 7.80 (m, 1H); 8.10 (m, 1H); 12.20 (s, 1H).

**Example 52**

7-(1-Adamantyl)-5-ethyl-2-(2-thienyl)imidazo[5,1-f][1,2,4]triazin4(3H)-one

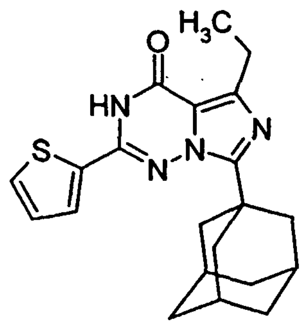

In analogy to the procedure for Example 1, 169 mg (0,42 mmol) of Example 79A, 329 mg (2,15 mmol) phosphoric trichloride are stirred at reflux for 3 hours, proportionate amounts of the solvents are used.
Yield: 20.5 mg (13%)
$^1$H-NMR (300 MHz, DMSO): δ = 1.20 (t, 3H); 1.80 (m, 6H); 2.10 (m, 3H); 2.25 (m, 6H); 2.80 (quart., 2H); 7.20 (m, 1H); 7.80 (m, 1H); 8.10 (m, 1H); 12.00 (s, 1H).

**Example 53 and Example 54**

7-(4-tert-Butylcyclohexyl)-5-ethyl-2-(3-pyridinyl)imidazo[5,1-f][1,2,4]triazin-4(3H)-one

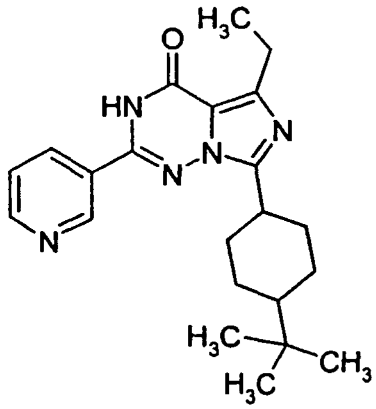

In analogy to the procedure for Example 1, 200 mg (0,50 mmol) of Example 80A, 165 mg (1,07 mmol) phosphoric trichloride are stirred at reflux for 3 hours, proportionate amounts of the solvents are used.
Yield: 7 mg (4%) cis-isomer
9 mg (5%) trans-isomer

cis-isomer (Example 53):
$^1$H-NMR ($CDCl_3$, 200 MHz): δ = 0.8 (s, 9H), 1.1 (m, 1H), 1.4 (t, 3H), 1.5-1.7 (m, 6H), 2.4 (m, 2H), 3.0 (q, 2H), 3.6 (m, 1H), 7.5 (m, 1H), 8.3 (m, 1H), 8.8 (m, 1H), 9.2 (s, 1H), 9.9 (s, 1H) ppm.

trans-isomer (Example 54):
$^1$H-NMR ($CDCl_3$, 200 MHz): δ = 0.8 (s, 9H), 1.2 (m, 3H), 1.3 (t, 3H), 1.8 (m, 4H), 2.1 (m, 2H), 3.0 (q, 2H), 3.2 (m, 1H), 7.5 (m, 1H), 8.3 (m, 1H), 8.8 (m, 1H), 9.3 (s, 1H), 10.2 (s, 1H) ppm.

**Example 55**

7-(4-cis-tert-Butylcyclohexyl)-5-ethyl-2-(2-pyridinyl)imidazo[5,1-f][1,2,4]triazin-4(3H)-one

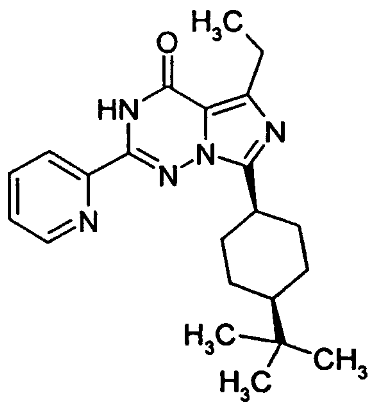

In analogy to the procedure for Example 1, 200 mg (0,50 mmol) of Example 81A, 165 mg (1,07 mmol) phosphoric trichloride are stirred at reflux for 3 hours, proportionate amounts of the solvents are used.
Yield: 44 mg (23%)
$^1$H-NMR ($d_6$-DMSO, 200 MHz): δ = 0.8 (s, 9H), 1.1 (m, 1H), 1.2 (t, 3H), 1.5-1.7 (m, 6H), 2.4 (m, 2H), 2.9 (q, 2H), 3.6 (m, 1H), 7.6 (m, 1H), 8.1 (m, 1H), 8.2 (m, 1H), 8.7 (m, 1H), 11.3 (s, 1H) ppm.

**Example 56**

7-(4-cis-tert-Butylcyclohexyl)-5-ethyl-2-(4-pyridinyl)imidazo[5,1-f][1,2,4]triazin-4(3H)-one

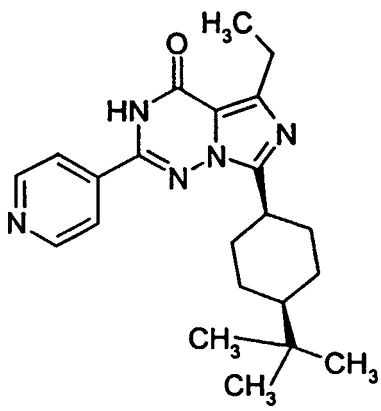

In analogy to the procedure for Example 1, 200 mg (0,42 mmol) of Example 82A, 165 mg (1,07 mmol) phosphoric trichloride are stirred at reflux for 3 hours, proportionate amounts of the solvents are used.
Yield: 9.6 mg (5%)
$^1$H-NMR ($d_6$-DMSO, 200 MHz): $\delta$ = 0.8 (s, 9H), 1.1 (m, 1H), 1.2 (t, 3H), 1.5-1.7 (m, 6H), 2.2 (m, 2H), 2.9 (q, 2H), 3.6 (m, 1H), 7.9 (m, 2H), 8.8 (m, 2H), 11.9 (s, 1H) ppm.

**Example 57**

7-(4-cis-tert-Butylcyclohexyl)-2-(2,5-dichloro-1,3-thiazol-4-yl)-5-ethylimidazo[5,1-f][1,2,4]triazin-4(3H)-one

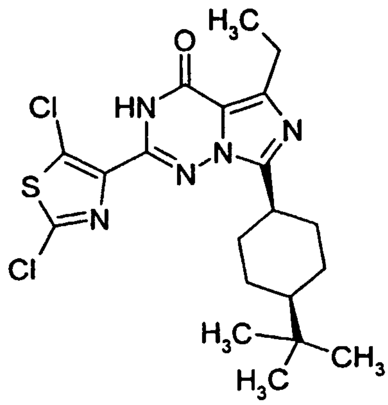

In analogy to the procedure for Example 1, 50 mg (0,11 mmol) of Example 83A, 165 mg (1,07 mmol) phosphoric trichloride are stirred at reflux for 3 hours, proportionate amounts of the solvents are used.
Yield: 11.7 mg (24%)
$^1$H-NMR ($d_6$-DMSO, 200 MHz): $\delta$ = 0.8 (s, 9H), 1.1 (m, 1H), 1.2 (t, 3H), 1.5-1.7 (m, 6H), 2.2 (m, 2H), 2.9 (q, 2H), 3.5 (m, 1H), 11.9 (s, 1H) ppm.

**Example 58**

7-(4-tert-Butylcyclohexyl)-5-ethyl-2-(2-furyl)imidazo[5,1-f][1,2,4]triazin-4(3H)-one

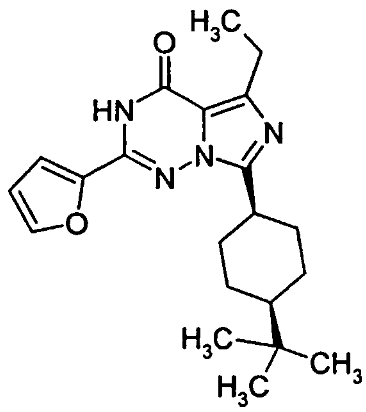

In analogy to the procedure for Example 1, 250 mg (0,65 mmol) of Example 84A, 250 mg (1,61 mmol) phosphoric trichloride are stirred at reflux for 3 hours, proportionate amounts of the solvents are used.
Yield: 67 mg (28%)
$^1$H-NMR ($d_6$-DMSO, 200 MHz): $\delta$ = 0.8 (s, 9H), 1.1 (m, 1H), 1.2 (t, 3H), 1.5-1.7 (m, 6H), 2.1 (m, 2H), 2.9 (q, 2H), 3.5 (m, 1H), 6.7 (m, 1H), 7.5 (m, 1H), 7.9 (m, 2H), 11.8 (s, 1H) ppm.

## Claims

1. Compounds of the general formula (I),

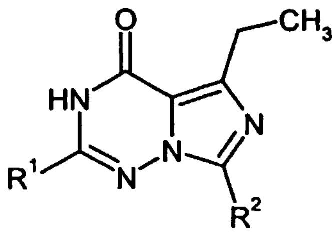

(I)

in which

$R^1$ denotes 5- to 10-membered heteroaryl, which is optionally substituted by identical or different residues selected from the group consisting of halogen, ($C_1$-$C_4$)-alkyl, trifluoromethyl, phenyl, cyano, nitro und trifluoromethoxy,

and

$R^2$ denotes 3- to 10-membered carbocyclyl or carbon-bonded, 4- to 10-membered heterocyclyl, whereby carbocyclyl and heterocyclyl are optionally substituted by identical or different residues selected from the group consisting of ($C_1$-$C_6$)-alkyl, ($C_1$-$C_6$)-alkoxy, hydroxy, halogen, trifluoromethyl and oxo,
or
denotes ($C_2$-$C_{10}$)-alkyl, which is optionally substituted by identical or different residues selected from the group consisting of ($C_1$-$C_6$)-alkoxy, hydroxy, halogen, 3- to 10-membered carbocyclyl and oxo,

and their salts, hydrates and/or solvates.

**2.** Compounds according to claim 1, whereby

$R^1$ denotes furanyl, thiophenyl, thiazolyl, pyridyl, chinolyl or isochinolyl, which are optionally substituted by identical or different residues selected from the group consisting of halogen, ($C_1$-$C_4$)-alkyl, trifluoromethyl, cyano, nitro und trifluoromethoxy.

**3.** Compounds according to claim 1 or 2, whereby

$R^2$ denotes ($C_4$-$C_7$)-cycloalkyl, which is optionally substituted up to two times by identical or different ($C_1$-$C_5$)-alkyl residues, or denotes ($C_3$-$C_8$)-alkyl, which is optionally substituted by a ($C_4$-$C_7$)-cycloalkyl.

**4.** A process for the preparation of the compounds according to claim 1, **characterized in that**, compounds of the general formula (IV),

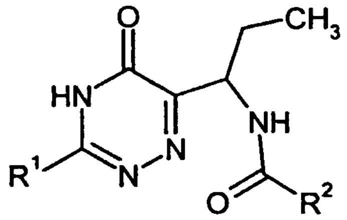

(IV)

in which $R^1$ and $R^2$ have the meaning indicated in claim 1, are reacted with a dehydrating agent.

**5.** Compounds of the general formula (IV) according to claim 4.

**6.** Compounds according to any one of claims I to 3 for therapeutic and/or prophylactic use.

**7.** Pharmaceutical composition containing at least one compound according to any one of claims 1 to 3 and a pharmacologically acceptable diluent.

**8.** Use of compounds according to any one of claims 1 to 3 for the preparation of medicaments.

**9.** Use of compounds according to any one of claims 1 to 3 for the preparation of medicaments for the treatment and/or prophylaxis of inflammatory processes and/or immune diseases.

**10.** Use of compounds according to any one of claims 1 to 3 for the preparation of medicaments for the treatment and/or prophylaxis of chronic obstructive pulmonary disease and/or asthma.

## Patentansprüche

**1.** Verbindungen der allgemeinen Formel (I):

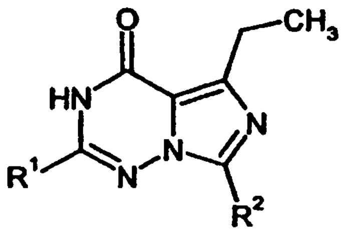

(I) ,

worin gilt:

$R^1$ bedeutet 5- bis 10-gliedriges Heteroaryl, das gegebenenfalls mit gleichen oder unterschiedlichen Resten substituiert ist, ausgewählt aus der Gruppe, bestehend aus Halogen, $(C_{1-4})$-Akyl, Trifluormethyl, Phenyl, Cyano, Nitro und aus Trifluormethoxy,
und
$R^2$ bedeutet 3- bis 10-gliedriges Carbocyclyl oder Kohlenstoffgebundenes 4- bis 10-gliedriges Heterocyclyl, wobei das Carbocyclyl und Heterocyclyl gegebenenfalls mit gleichen oder unterschiedlichen Resten substituiert sind, ausgewählt aus der Gruppe, bestehend aus $(C_{1-6})$-Alkyl, $(C_{1-6})$-Alkoxy, Hydroxy, Halogen, Trifluormethyl und aus Oxo, oder
es bedeutet $(C_{2-10})$-Alkyl, das gegebenenfalls mit gleichen oder unterschiedlichen Resten substituiert ist, ausgewählt aus der Gruppe, bestehend aus $(C_{1-6})$-Alkoxy, Hydroxy, Halogen, 3- bis 10-gliedrigem Carbocyclyl und aus Oxo,

sowie deren Salze, Hydrate und/oder Solvate.

**2.** Verbindungen gemäß Anspruch 1, in denen
$R^1$ Furanyl, Thiophenyl, Thiazolyl, Pyridyl, Chinolyl oder Isochinolyl bedeutet, die gegebenenfalls mit gleichen oder unterschiedlichen Resten substituiert sind, ausgewählt aus der Gruppe, bestehend aus Halogen, $(C_{1-4})$-Alkyl, Trifluormethyl, Cyano, Nitro und aus Trifluormethoxy.

**3.** Verbindungen gemäß Anspruch 1 oder 2, in denen
$R^2$ $(C_{4-7})$-Cycloalkyl, das gegebenenfalls mit bis zu 2 gleichen oder unterschiedlichen $(C_{1-5})$-Alkylresten substituiert ist, oder $(C_{3-8})$-Alkyl bedeutet, das gegebenenfalls mit $(C_{4-7})$-Cycloalkyl substituiert ist.

**4.** Verfahren zur Herstellung der Verbindungen gemäß Anspruch 1, **dadurch gekennzeichnet, dass** Verbindungen der allgemeinen Formel (IV):

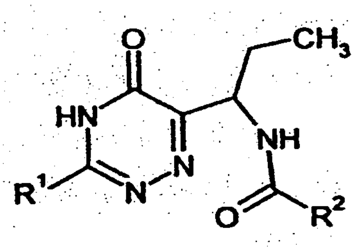

(IV) ,

in denen $R^1$ und $R^2$ die in Anspruch 1 angegebene Bedeutung haben, mit einem wasserabspaltenden Mittel zur Reaktion gebracht werden.

**5.** Verbindungen der allgemeinen Formel (IV) gemäß Anspruch 4.

**6.** Verbindungen gemäß einem der Ansprüche 1 bis 3 zur therapeutischen und/oder prophylaktischen Anwendung.

**7.** Pharmazeutische Zusammensetzung, enthaltend mindestens eine Verbindung gemäß einem der Ansprüche 1 bis 3 und ein pharmakologisch zulässiges Verdünnungsmittel.

**8.** Verwendung von Verbindungen gemäß einem der Ansprüche 1 bis 3 zur Herstellung von Medikamenten.

**9.** Verwendung von Verbindungen gemäß einem der Ansprüche 1 bis 3 zur Herstellung von Medikamenten zur Behandlung und/oder Prophylaxe von entzündlichen Prozessen und/oder von Immunkrankheiten.

**10.** Verwendung von Verbindungen gemäß einem der Ansprüche 1 bis 3 zur Herstellung von Medikamenten zur Behandlung und/oder Prophylaxe von chronischer zerstörerischer Lungenkrankheit und/oder von Asthma.

## Revendications

**1.** Composés de formule générale (I),

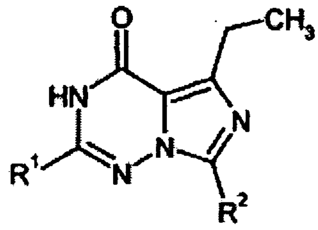

(I)

dans laquelle

$R^1$ désigne un reste hétéroaryle pentagonal à décagonal, qui est facultativement substitué par des résidus identiques ou différents choisis dans le groupe consistant en halogène, alkyle en $C_1$ à $C_4$, trifluorométhyle, phényle, cyano, nitro et trifluorométhoxy,

et

$R^2$ désigne un reste carbocyclyle trigonal à décagonal ou un reste hétérocyclyle tétragonal à décagonal lié par le carbone, les restes carbocyclyle et hétérocyclyle étant facultativement substitués par des résidus identiques ou différents choisis dans le groupe consistant en alkyle en $C_1$ à $C_6$, alkoxy en $C_1$ à $C_6$, hydroxy, halogène, trifluorométhyle et oxo,
ou
désigne un reste alkyle en $C_2$ à $C_{10}$, qui est facultativement substitué par des résidus identiques ou différents choisis dans le groupe consistant en alkoxy en $C_1$ à $C_6$, hydroxy, halogène, carbocyclyle trigonal à décagonal et oxo,

et leurs sels, leurs hydrates et/ou leurs produits de solvatation.

**2.** Composés suivant la revendication 1, dans lesquels

$R^1$ désigne des restes furannyle, thiophényle, thiazolyle, pyridyle, quinolyle ou isoquinolyle, qui sont facultativement substitués par des résidus identiques ou différents choisis dans le groupe consistant en halogène, alkyle en $C_1$ à $C_4$, trifluorométhyle, cyano, nitro et trifluorométhoxy.

**3.** Composés suivant la revendication 1 ou 2, dans lesquels

$R^2$ désigne un reste cycloalkyle en $C_4$ à $C_7$, qui est facultativement substitué jusqu'à deux fois par des résidus alkyle en $C_1$ à $C_5$ identiques ou différents, ou désigne un reste alkyle en $C_3$ à $C_8$ qui est facultativement substitué par un résidu cycloalkyle en $C_4$ à $C_7$.

**4.** Procédé de production des composés suivant la revendication 1, **caractérisé en ce que** des composés de formule générale (IV),

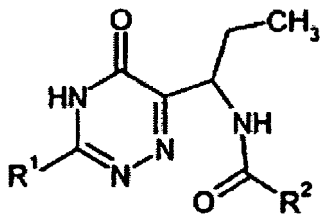

(IV)

dans laquelle $R^1$ et $R^2$ ont les définitions indiquées dans la revendication 1, sont amenés à réagir avec un agent déshydratant.

**5.** Composés de formule générale (IV) suivant la revendication 4.

6. Composés suivant l'une quelconque des revendications 1 à 3, destinés à un usage thérapeutique et/ou prophylactique.

7. Composition pharmaceutique contenant au moins un composé selon l'une des revendications 1 à 3 et un diluant pharmaceutiquement acceptable.

8. Utilisation de composés suivant l'une quelconque des revendications 1 à 3 pour la préparation de médicaments.

9. Utilisation de composés suivant l'une quelconque des revendications 1 à 3 pour la préparation de médicaments destinés au traitement et/ou à la prophylaxie de processus inflammatoires et/ou de maladies immunitaires.

10. Utilisation de composés suivant l'une quelconque des revendications 1 à 3 pour la préparation de médicaments destinés au traitement et/ou à la prophylaxie de la bronchopneumopathie chronique obstructive et/ou de l'asthme.